# EUROPEAN PATENT SPECIFICATION

(11) **EP 1 267 905 B1**
(45) Date of publication and mention of the grant of the patent: **28.09.2005**
(21) Application number: 01911868.6
(22) Date of filing: 09.03.2001
(51) Int. Cl.: A61K 39/395, A61P 9/00

(54) **COMPOSITION FOR THE TREATMENT OF HEART FAILURE**
ZUSAMMENSETZUNG ZUR BEHANDLUNG VON HERZINSUFFIZIENZ
COMPOSITION DESTINEE AU TRAITEMENT DE L'INSUFFISANCE CARDIAQUE

(30) Priority: 10.03.2000 GB 0005867; 13.11.2000 GB 0027661
(43) Date of publication of application: 02.01.2003
(73) Proprietor: MEDINNOVA AS, 0027 Oslo (NO)
(72) Inventor: AUKRUST, Pal, N-0027 Oslo (NO); FROLAND, Stig, N-0027 Oslo (NO); SIMONSEN, Svein, N-0027 Oslo (NO); AASS, Halfdan, N-0027 Oslo (NO); FJELD, Jan, N-0027 Oslo (NO); ANDREASSEN, Arne, N-0027 Oslo (NO); IHLEN, Halfdan, N-0027 Oslo (NO); KJEKSHUS, John, N-0027 Oslo (NO); NITTER-HAUGE, Sigurd, N-0027 Oslo (NO); UELAND, Thor, N-0027 Oslo (NO); GULLESTAD, Lars, N-Baerum Postterminal (NO); LIEN, Egil, N-7491 Trondheim (NO)
(74) Representative: Dzieglewska, Hanna Eva
(86) International application number: PCT/GB2001/001054
(87) International publication number: WO 2001/066124

(56) References cited:
- WO-A-94/28932
- WO-A-95/24927
- WO-A-99/64462
- US-A- 4 800 078
- GREIBER S. ET AL.: "Intensive medicine treatment of the Guillain-Barré-Syndrome" INTENSIVMEDIZIN UND NOTFALLMEDIZIN, vol. 35, no. 1, 1998, pages 50-55, XP001021585
- BIESERT L., ET AL.: "Viral safety of a new polyvalent intravenous immunoglobulin (octagam)" ANNALS OF HEMATOLOGY, vol. 70, no. no. suppl. , 1995, page a84 XP001021593

## Description

The present invention relates to heart disorders and in particular the use of immunomodulating agents for the treatment of heart disorders and the symptoms associated therewith.

Heart disorders or diseases, which are generally characterised by impaired cardiac function, e.g. heart failure affect a large number of people worldwide and in particular in the Western world. Heart disorders or diseases are responsible for a reduced quality of life and premature death in a significant proportion of sufferers. Heart disorders occur in men, women, and children of both sexes, but are particularly prevalent in men and in elderly or middle aged people.

Heart failure is characterized by impaired cardiac function either due to reduced pump function (systolic dysfunction) or reduced filling (diastolic dysfunction). There are a number of different causes of heart failure of which the most common in the western world is coronary artery disease. Other common causes are cardiomyopathy (primary or secondary), hypertension, valvular diseases, and congenital defects.

Approximately 70% of heart failure in the western world are caused by coronary artery disease, which is usually due to atherosclerosis. Atherosclerosis will result in narrowing of the vessels in the heart leading to inadequate blood supply to the myocardium (muscle cells). Such heart disorders which involve a reduced supply of blood to the heart are sometimes given the general term "ischemic heart disease". Ischemic heart disease (or ischemic cardiomyopathy) is the major etiologic group of heart failure in the Western world.

A reduced blood supply to the heart can manifest itself as angina pectoris (pain in the chest), acute myocardial infarction (which is the result of acute coronary artery occlusion causing a damaged myocardium with scar tissue; such an area cannot sustain cardiac muscle function), and sudden deaths. If the blood supply to the heart is reduced over periods of weeks to years, or if the myocardium has been substantially weakened by infarction with scar tissue, the heart function will become weakened with reduced pumping ability leading to the clinical manifestation of chronic heart failure.

Clearly, with heart disease being so common worldwide, much effort has been associated with trying to develop treatments and therapies. Non pharmacological treatments include reduction of sodium intake, a modified diet, weight loss and controlled exercise programmes. In more serious cases heart disorders may be treated by surgical means, for example coronary bypass surgery, coronary angioplasty or even transplantation.

A number of pharmaceutical treatments are available and are well known and documented in the art. Such treatments for example involve the use of diuretics, vasodilators, inotropic drugs such as digoxin, or other compounds such as anticoagulants, β blockers or angiotensin converting enzyme (ACE) inhibitors.

Although treatment has been substantially improved during recent years the mortality and morbidity for heart failure is substantial. While annual mortality is from 5 to 50% (analogous to that of many cancers) the hospital admissions rate is high and is the single most common cause of admissions in UK and US.

Thus, there is a continued demand for new, non toxic, treatment regimes which can be used to treat heart disorders.

Immunomodulating agents, for example corticosteroids and, more recently, preparations of IVIG (intravenous immunoglobulins) have been used to treat a number of conditions thought to involve the immune system in its pathogenesis. However, much of the data and studies which have been documented to date using IVIG have not been carried out in a controlled way and, moreover, some of the results are conflicting. Thus, although by 1992 IVIG therapy had been reported to be beneficial for more than 35 diseases thought to be produced by immunopathology (e.g. immunohematologic disorders and nonhematologic autoimmune diseases), convincing results for the use of IVIG as a therapy had been presented for only a handful of diseases including acute autoimmune thrombocytopenic purpora of childhood and Kawasaki's syndrome (see review of Dwyer. J., The New England Journal of Medicine, Vol. 326 (2), 107-116, 1993).

Thus, it can be seen that the use of IVIG in therapy has focussed on diseases associated with an immunopathology, e.g. an autoimmune/viral pathogenesis.

Indeed, more recently IVIG has been shown to be potentially effective in the treatment of two types of heart failure which are thought to be caused by viral infection and associated with an autoimmune/viral pathogenesis. Thus, IVIG has been proposed to be useful in the treatment of children (and adults) with new onset (acute) dilated cardiomyopathies (Drucker et al., 1994, Circulation, Vol 89, 252-257 and McNamara et al., 1997, Circulation, Vol 95, 2476-2478) and also in the treatment of acute myocarditis (McNamara, supra). Although perhaps obvious candidates for IVIG therapy, the initial attempts to abrogate the development of such diseases by immunosuppressive therapy were however unsuccessful (Parrillo et al., N. Engl. J. Med. 1989; 321, 1061-1068 and Mason et al., N. Engl. J. Med. 1995; 333, 269-275) and even though effective results have now been obtained by McNamara and Drucker, these involved only a small number of patients in non placebo controlled trials.

Surprisingly, it has now been found that immunomodulating agents are effective in the treatment of heart disorders which are not necessarily induced by or connected with a viral or autoimmune pathogenesis. Moreover, such agents are also effective to treat the symptoms associated with said heart disorders.

Thus, in one aspect the present invention provides the use of an immunomodulating agent being intravenous immunoglobulin (IVIG) in the manufacture of a medicament for use in the treatment of non-viral or non-autoimmune induced heart disorders or non-viral or non-autoimmune associated phases of heart disorders, wherein said heart disorder is an ischaemic heart disease or a chronic non-ischaemic cardiomyopathy.

A further aspect of the present invention provides the use of an intravenous immunoglobulin (IVIG) in the manufacture of a medicament for use in the treatment or alleviation of symptoms associated with such heart disorders or phases.

"Non-viral or non-autoimmune induced heart disorders" as used herein refers to heart disorders or diseases in which the impaired cardiac function associated with the disorder does not occur as a direct result of a viral infection and/or an autoimmune based reaction.

Typical examples of such diseases are ischemic heart diseases such as angina pectoris, acute coronary syndromes (unstable angina pectoris and acute myocardial infarction) and chronic congestive heart failure (CHF or cCHF). The main subgroup of cCHF is chronic ischemic heart disease, i.e. chronic ischemic cardiomyopathy. Another subgroup of cCHF includes chronic non-ischemic cardiomyopathy including idiopathic dilated cardiomyopathy, and cardiomyopathy due to hypertension, valvular disease or congenital defects. Coronary artery disease (CAD) may lead to several clinical consequences: development of stable angina pectoris, acute coronary syndromes (unstable angina pectoris and acute myocardial infarction) and chronic ischemic heart failure or cardiomyopathy. Thus CAD may lead to cCHF (i.e. chronic ischemic cardiomyopathy), but is clearly not identical to cCHF. Thus, stable angina and acute coronary syndromes are not included in the cCHF group. Another example of such a disease is transplant coronary artery disease (a chronic condition of the transplanted heart).

On the other hand, classic examples of diseases which are caused by viral infection and possibly an autoimmune based reaction are acute myocarditis and acute dilated cardiomyopathy and these diseases are excluded from this definition.

Some heart diseases are thought to have several stages or phases, some of which are a direct result of a viral infection or an autoimmune based reaction and some of which are not. For example, acute heart failure may be due to a viral infection. This may result in complete normalization of the heart or could be followed by a chronic phase characterized by stable but reduced myocardial function or progressive deterioration in myocardial function with increasing symptoms of heart failure. It is likely that this chronic phase is not directly the result of viral damage, but is due to neuroendocrine factors, growth factors and prolonged inflammatory responses to the viral infection. The treatment of such non-viral or non-autoimmune associated phases of heart diseases is included in the scope of the present invention. However, it should be noted that a common cause of acute CHF is heart failure during acute myocardial infarction or acute exacerbation of chronic heart failure. Thus, in many cases chronic heart failure has no association with a viral infection at any stage in its development.

Preferably heart disorders to be treated according to the present invention are chronic CHF, chronic (stable) angina pectoris, or acute coronary syndromes. The treatments of the present invention are effective to treat chronic CHF, chronic (stable) angina pectoris, acute coronary syndromes, or the symptoms thereof regardless of the cause of disease.

Stable angina pectoris is a discomfort in the chest caused by myocardial ischemia brought on by exertion and associated with a disturbance of myocardial function but without myocardial necrosis. The underlying cause of angina pectoris is usually coronary artery disease caused by atherosclerosis. Atherosclerosis is a generic term for a number of diseases in which the arterial wall becomes thickened and looses elasticity. It usually starts in childhood with clinical manifestations (angina pectoris, acute coronary syndromes, congestive heart failure, sudden death, brain infarction, and peripheral artery disease) in middle to late adulthood.

Acute coronary syndromes to be treated in accordance with the present invention include unstable angina pectoris, non-Q wave myocardial infarction and Q-wave myocardial infarction. These syndromes are believed to be the common pathophysiological substrate caused by a rupture of an atherosclerotic plaque in one of the coronary arteries. (When a plaque ruptures, a sufficient quantity of thrombogenic substances are exposed leading to thrombus formation and obstruction of the coronary lumen).

As alluded to briefly above, heart failure is a common disorder of the heart and can be defined as a disorder which may result from any condition that reduces the ability of the heart to pump blood. Often the cause is decreased contractility of the myocardium resulting from diminished coronary blood flow (e.g. heart failure caused by coronary ischemic disease), but failure to pump adequate quantities of blood can also be caused by damage to heart valves, external pressure around the heart, primary cardiac muscle diseases (e.g. idiopathic dilated cardiomyopathy) or any other abnormality which makes the heart a hypoeffective pump.

Cardiac failure may be manifest in either of two ways: (1) by a decrease in cardiac output or (2) by a damming of blood in the veins behind the left or right heart. The heart can fail as a whole unit or either the left side or the right side can fail independently of the other. Either way this type of heart failure leads to circulatory congestion and, as a result is referred to a congestive heart failure (CHF).

Congestive heart failure can be divided into two phases, acute (short term and unstable) CHF and chronic (long term and relatively stable) CHF. The division between the two is difficult to define precisely, but generally acute CHF is the stage of failure which occurs immediately after heart damage (i.e. has a rapid onset and short course) and is associated with instability in cardiac function and circulation, for example a sudden drop in cardiac output. Providing the acute phase is not so severe as to result in death, the sympathetic reflexes of the body are immediately activated and can compensate for the sudden loss in cardiac function. Such compensation can often be so effective and rapid that it is possible that no noticeable effect on the patient will be felt if a patient remains calm.

After the first few minutes of an acute heart attack, a prolonged secondary state begins. This is characterised by a retention of fluid by the kidneys and by the progressive recovery of the heart over a period of several weeks to months up until the point at which the cardiac condition stabilises. This phase of stability is known as chronic CHF. Although the heart has compensated and stabilised it is still weak and may become progressively weaker.

This means therefore that although symptoms vary largely between patients, patients with chronic CHF characteristically have a reduced cardiac function. The most common manifestation of reduced cardiac performance is systolic dysfunction. For example such patients display a reduced left ventricular ejection fraction (LVEF) when compared to a "normal" person who has not suffered from heart failure. In normal persons left ventricular ejection fraction is usually above 60%, while an ejection fraction less than 40% is characterized as systolic dysfunction. Thus, a LVEF of less than 40% is characteristic of reduced heart function in patients with chronic CHF. Less common than systolic dysfunction is diastolic dysfunction in which the ejection fraction is relatively preserved (left ventricular EF>40%) or normal, but where left ventricular filling is reduced.

Other characteristics of reduced cardiac function such as for example a reduced right ventricular ejection fraction, reduced exercise capacity and impaired haemodynamic variables such as a decreased cardiac output, increased pulmonary arterial pressure and increased heart rate and low blood pressure are also often observed in patients with cCHF.

The New York Heart Association (NYHA) classification system divides heart disease into four classes, depending on the severity of disease. NYHA class I: Patient with cardiac disease but without resulting limitations of physical activity; Class II: Patient with cardiac disease resulting in slight limitation of physical activity. Class III: Patient with cardiac disease resulting in marked limitation of physical performance. They are comfortable at rest. Class IV. Patient with cardiac disease resulting in inability to carry on any physical activity without discomfort. Symptoms may be present at rest. Immunomodulating agents are suitable for the treatment of all classes of heart failure but particularly the classes II-IV and more preferably the classes II-III on the NYHA classification scale.

Thus, while the acute phase of CHF is over relatively quickly the stability associated with the chronic phase of CHF can take a matter of months to develop. Generally, a patient exhibiting symptoms of CHF for greater than 3 months or more preferably greater than 6 months can be regarded as having chronic stable CHF, providing that no further symptoms of acute congestive heart failure such as angina or evidence of myocardial infarction have occurred during this 3 month or 6 month period.

As was also alluded to briefly above, the chronic CHF to be treated according to the present invention may result from any cause, e.g. may be the result of a primary disease or may be secondary to another disease. In a preferred embodiment of the invention the chronic CHF to be treated is secondary to either idiopathic dilated cardiomyopathy (IDCM) and/or coronary ischemic disease (coronary artery disease - CAD).

As indicated above, a further aspect of the invention, lies in the use of IVIG as an immunomodulating agent in the treatment of acute coronary syndromes. Examples of acute coronary syndromes which may be treated or have their symptoms alleviated in accordance with the present invention are unstable angina pectoris, myocardial infarction (e.g. non-Q wave and Q-wave myocardial infarction). These syndromes are believed to have a common pathophysiological substrate caused by a rupture of an atherosclerotic plaque in one of the coronary arteries. (When a plaque ruptures, a sufficient quantity of thrombogenic substances are exposed leading to thrombus formation and obstruction of the coronary lumen). If the lumen is completely obstructed this typically causes necrosis of the myocardium (i.e. transmural myocardial infarction often with Q-wave development on the ECG). Less obstructive thrombi produce unstable angina pectoris and non-Q-wave infarction typically presenting as ST-segment changes on ECG, with non-Q infarction presenting with biochemical markers of myocardial necrosis in addition.

"Immunomodulating agent" as used herein refers to any compound, agent or composition which alters or effects any component of the immune system. Such alteration may be an increase or decrease in the amount or activity of a component(s) of the immune system as compared to the amount or activity of the same component(s) in an untreated individual. The component(s) effected may be components of the innate (humoral) or cellular immune system. For example, an immunomodulating agent might effect the immune system by way of stimulating the increased or reduced expression or activity of certain cytokines, cytokine receptors or cytokine agonists/antagonists, which are involved in an inflammatory response. Cytokines, cytokine receptors or cytokine agonists/antagonists are often either pro-inflammatory (e.g. IL-1, TGFα, IL-6) or anti-inflammatory (e.g. IL-10, sTNFRs, IL-IRa). An immunomodulating agent might also effect the immune system by modulating an increased or reduced expression of sub-sets of cytokines, for example chemokines or their receptors. Chemokines are a new sub-set of inflammatory cytokines which were discovered relatively recently and have some important common properties. Such properties include for example the induction of directed migration of leukocytes into inflamed tissue. In addition chemokines are thought to be involved in the regulation of other leukocyte properties such as proliferation, induction of cytokines, stimulation of enzyme secretion and oxidative stress.

Alternatively or additionally the immunomodulating agent may alter the function of the cellular arm of the immune system, i.e. alter the activity of T cells and/or B cells, either via the modulation of the cytokine or chemokine network or by some other mechanism. Finally, the immunomodulating agent may induce, reduce or inhibit the activation of complement or the functioning of other cells or components associated with the immune system, e.g. scavenger cells (phagocytes), leukocytes, endothelial cells and cell adhesion molecules.

The immunomodulating agent for use in the present invention is intravenous immunoglobulin (IVIG or IVIg). IVIG is a preparation of immunoglobulins which is produced from plasma. For example, IVIG is typically a virus-inactivated IVIG preparation (PH 4) produced from fresh frozen plasma collected in blood banks. The final product may be dispensed in sterile water containing for example 10% maltose. The preparation contains mainly IgG. For example a typical preparation contains a final IgG concentration of at or around 5 g/L and has less than 0.1 g/L IgA and IgM). Ideally the preparation will contain minimal or undetectable levels of cytokine and cytokine inhibitors. Detection of such cytokines etc. can be carried out using standard enzyme immunoassays and ideally no IL-1β, IL-1 receptor antagonist, TNFα, soluble TNF receptors or IL-10 will be detected in the IVIG product. In addition, preferably the endotoxin levels in the IVIG preparation will be low, e.g. less than 10 pg/mL. Several IVIG preparations suitable for use in the present invention are available commercially, such as Octagam from Octapharma.

Other immunomodulating agents are known and described in the art. These include traditional immunomodulating agents such as for example corticosteroids, nucleotide analogs and cyklosporine. In addition, cytokine inhibitors such as IL-1Ra, p75TNFR and pentoxifylline, or cytokines such as IL-10 may be mentioned.

The IVIG immunomodulating agent for use in accordance with the present invention has the effect of producing a measurable and preferably significant improvement in the heart disorder or its associated symptoms. Preferably, the immunomodulating agent will produce an improved quality of life for a patient and, ultimately a prolonged lifetime.

Given the nature of most forms of heart disease it is not to be expected that "treatment" in accordance with the present invention will result in a complete cure of the heart disorder in question. Rather, "treatment" in accordance with the present invention should include an improvement or alleviation of any of the symptoms associated with heart disorders and also an improved quality of life for a patient and, ultimately a prolonged lifetime and improved survival. "Treatment" in accordance with the present invention also includes an improvement or increase of the functionality of the heart or, in other words an improvement or increase in cardiac function or performance. In particular, treatment in accordance with the present invention may result in an improvement or increase in any one or more of the symptoms and functional parameters associated with heart disorder patients and in particular the following symptoms and parameters of the patient.

The first symptom and parameter associated with improved cardiac function in heart disorder patients is an increase in ventricular ejection fraction and in particular left ventricular ejection fraction (LVEF). This can be assessed by standard methods well known and documented in the art, for example by echocardiography, ECG synchronized gated radionuclide ventriculography (MUGA scan), angiography or magnetic resonance (MR) imaging, and is normally carried out when the subject is at rest. In studies carried out in accordance with the present invention, patients with an LVEF of less than 40% have shown a significant improvement in LVEF (by up to 5%) during the course of treatment with the immunomodulator IVIG. An improvement in LVEF has been found to be associated with improved survival amongst CHF patients (Cintron et al., 1993, Circulation Vol 87, Supplement VI, 17-23). Thus, this is an important and advantageous parameter to be improved in subjects treated in accordance with the present invention. RVEF was also significantly increased.

Whilst an improvement of LVEF is particularly important for the overall improvement of heart function, a number of other parameters associated with cardiac performance are improved in accordance with the present invention. One of these is a significant improvement in overall clinical status and thus clinical performance as evaluated by NYHA functional class. In other words the NYHA functional class of a patient will be reduced after treatment with immunomodulating agents in accordance with the present invention. Such a clinical evaluation may normally be carried out by a trained cardiologist.

Also an improvement was seen in exercise capacity of the patients, as measured by peak oxygen uptake and peak work load. As indicated above, a decreased exercise capacity is an inconvenient and potentially dehabilitating symptom of many heart disorder patients. Methods for measuring exercise capacity are well known and documented in the art. For example exercise testing can be carried out using an electrically braked bicycle ergometer. An exemplary protocol might consist of a starting work rate of 20 W increasing by 20 W every second minutes until exhaustion (defined as an inability to keep the pedalling rate steady at 60 rpm). Oxygen uptake (VO₂) can be measured using for example the EOS/SPRINT system. Peak VO₂, is taken as the highest VO₂ observed.

Furthermore various improvements in hemodynamic status and echocardiographic variables were also observed. These are again indicative of improved cardiac function. For example a significant decrease in pulmonary capillary wedge pressure and in pulmonary artery pressure was observed, together with an increase in peak heart rate, peak systolic blood pressure and mitral velocity deceleration time. Echocardiographic variables may conveniently be measured by echocardiography carried out by a trained cardiologist and haemodynamic variables can conveniently be assessed by right-sided heart catheterization according to standard techniques.

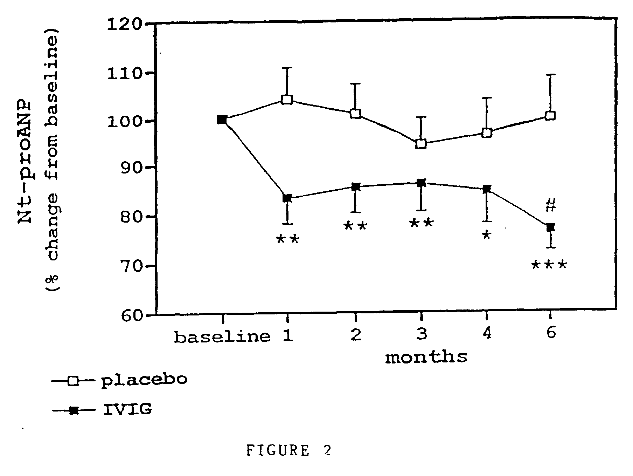

Another important variable which was assessed was the plasma level of Nt-proANP. An increased or generally high level of Nt-proANP has been recognised as a marker of cardiac disfunction. Moreover, levels of Nt-proANP have been shown in the past to correlate with pulmonary artery pressures in CHF and provide important prognostic information in CHF patients (Gottlieb et al., J. Am. Coll. Cardiol. 1989; 13: 1534-1539). In patients treated in accordance with the present invention the plasma levels of Nt-proANP decreased significantly during IVIG therapy. Moreover, the initial decrease was observed very early on in the therapy, i.e. after the initial induction therapy (see below) and the decrease then continued throughout the treatment period (Figure 2) suggesting that an extended treatment period may well result in even further decreases of this factor. Levels of Nt-proANP in a blood sample can be measured in a number of ways well known and documented in the art, for example by radioimmunoassay. Prior to the immunoassay, plasma is separated from a blood sample taken from the patient again by methods well known and documented in the art.

The above described "improvement" or "increase" in the symptoms and parameters includes any measurable improvement or increase when the parameter in question is compared with the equivalent parameter in a non-treated individual or when the parameter in question is compared with the equivalent parameter in the same individual taken at an earlier time point (e.g. comparison with a "base line" level). Preferably the improvement or increase will be a statistically significant one. Especially preferably the improvement or increase in the symptoms and parameters will be associated with the improved health of the patient concerned and more preferably a prolonged survival.

Methods of determining the statistical significance of differences in parameters are well known and documented in the art. For example herein a parameter is generally regarded as significant if a statistical comparison using a two-tailed significance test such as a Student t-test or Mann-Whitney U Rank-Sum test shows a probability value of <0.05.

Thus, a further aspect of the present invention relates to the use of IVIG as defined herein in the manufacture of a medicament for use in the improvement of cardiac function.

In a yet further and more preferred aspect, the present invention relates to the use of IVIG as defined herein in the manufacture of a medicament for use in the increase of ventricular function and particularly preferably left ventricular function (e.g. LVEF).

A yet further aspect of the present invention relates to the use of IVIG as defined above in the manufacture of a medicament for use in the reduction of plasma Nt-proANP levels. As mentioned above, a reduction in the plasma levels of Nt-proANP is an indicator of improved cardiac function and performance. "Reduction" as used herein includes any measurable reduction when the parameter in question is compared with the equivalent parameter in a non-treated individual or when the parameter in question is compared with the equivalent parameter in the same individual taken at an earlier time point (e.g. comparison with a "base line" level). Preferably the reduction is statistically significant as discussed above. Especially preferably the reduction in the levels of Nt-proANP will be associated with an improved feeling of health in the patient concerned and more preferably a prolonged survival.

An important observation in the present invention was that an analysis of cytokines in the blood samples of subjects showed significant changes in the levels of cytokines between treated and non-treated patients, which would appear to indicate a general but profound down regulation of immune activation in treated patients. In particular treatment in accordance with the present invention was associated with enhanced levels of the anti-inflammatory cytokines IL-10, IL-1Ra and sTNFRs as well as decreased levels of IL-1β/IL-1Ra and TNFα/sTNFR ratios. In treated subjects a strong positive correlation was also observed between the changes in IL-1Ra, IL-10, sTNFRs and the change in LVEF. Accordingly it appears that the anti-inflammatory effect of treatment may well be important for the treatment of the heart disorders and symptoms thereof in accordance with the present invention.

In addition, an analysis of chemokine levels in the blood samples of subjects showed significant changes in the levels of chemokines and chemokine receptors between treated and non-treated patients. In general, in comparison with healthy individuals it was observed that patients with CHF showed an up-regulation in chemokines and their receptors thought to be involved in an inflammatory response, and a down-regulation in chemokine receptors thought to be involved in lymphocyte homing. In other words, as for the cytokines, the levels of chemokines and their receptors observed in CHF patients appear to indicate a general but profound up-regulation of an inflammatory response. In particular, it was observed that CHF patients showed (i) markedly raised gene expression of the chemokines MIP-1α, MIP-1β and IL-8, (ii) enhanced gene expression of several chemokine receptors (i.e. CCR1, CCR2, CCR5, CXCR1, CXCR2 and CX₃CR), and (iii) reduced gene expression of chemokine receptors thought to be involved in constitutive lymphocyte homing (i.e. CCR7 and CXCR5).

Importantly it has been shown that treatment of CHF patients with IVIg induces a modulation of the expression of chemokines and chemokine receptors in the direction observed in non-CHF patients. For example, IVIG treated patients have been shown to have reduced levels, i.e. down-regulation of "inflammatory" chemokines and their receptors such as MIP-1α, MIP-1β and IL-8 and the receptors CCR1, CCR2, CCR5, CXCR1, CXCR2 and CX₃CR and increased levels, i.e. up-regulation of the chemokine receptors which are thought to increase lymphocyte homing e.g. CCR7 and CXCR5. Such modulation is not shown in patients which are untreated with IVIG (e.g. in placebo treated patients). In other words, IVIG has been demonstrated to restore the imbalance in the chemokine network which is observed in CHF. Furthermore, the down-regulation of the chemokines MIP-1α and MIP-1β induced by IVIG has been shown to have a positive correlation with increased LVEF, an important indicator of improved cardiac function (as discussed above).

Thus preferably the immunomodulating agent for use in the present invention will have the effect of down regulating immune activation and/or down regulating inflammation processes e.g. by reducing the levels or activity of one or more pro-inflammatory cytokines or their receptors such as IL-1β, TNFα, IL-1 and/or increasing the level or enhancing the activity of one or more anti-inflammatory mediators such as IL-10, IL-IRa and sTNFRs and/or reducing the levels or activity of one or more pro-inflammatory chemokines such as MIP-1α, MIP-1β, and IL-8 or the chemokine receptors CCR1, CCR2, CCR5, CXCR1, CXCR2 and CX₃CR, and/or increasing the levels or activity of one or more chemokines or their receptors which are involved in the reduction of an inflammatory response (i.e. "anti-inflammatory" chemokines), such as CCR7 and CXCR5 which are thought to be involved in lymphocyte homing. In addition, the ratio of TNFα/sTNFR (soluble tumour necrosis factor receptor) and IL-1β/IL-1Ra or any other ratios of cytokines or chemokines and their receptors which reflect the balance between pro-inflammatory and anti-inflammatory mediators may be altered in such a way as to have the general effect of down regulating the inflammation processes.

The above described "down-regulation", "reduction", "up-regulation", increase", "enhanced" levels or activity of chemokines, cytokines and their receptors includes any measurable change in level of the entity in question when the entity in question is compared with the levels or activity of the equivalent entity in a non-treated individual or when the level or activity of the entity in question is compared with the equivalent entity in the same individual taken at an earlier time point (e.g. comparison with a "base line" level). Preferably the improvement or increase will be a statistically significant one. Especially preferably the change in level of the entity in question will be associated with the improved health of the patient concerned and more preferably a prolonged survival.

Methods of determining the statistical significance of differences in parameters are well known and documented in the art. For example herein a change in level is generally regarded as significant if a statistical comparison using a two-tailed significance test such as a Student t-test or Mann-Whitney U Rank-Sum test shows a probability value of <0.05.

Accordingly a yet further aspect of the invention provides the use of IVIG in the manufacture of a medicament for use in reducing immune activation and/or inflammation processes in non-viral or non-autoimmune induced heart disorders as defined above.

The above discussed results indicate that abnormal expression and/or activation of both chemokines and cytokines (or their receptors) play a role in the pathogenesis of CHF. In addition, it seems that the immunomodulating agent IVIG is capable of restoring imbalance in the chemokine and cytokine network which is induced in CHF and that this modulation of the chemokine and cytokine network may represent an important mode of action of IVIG.

The capacity of IVIG to restore imbalance in the chemokine and/or cytokine network in CHF patients indicates that IVIg might be used to treat any disease in which such an imbalance in the chemokine or cytokine network was observed, for example other cardiovascular diseases and diseases involving inflammation. IVIG may be used in the manufacture of a medicament for use in the treatment of a disease which involves an imbalance in the chemokine and/or cytokine network and in the manufacture of a medicament for the modulation of and/or restoring imbalance in the chemokine and/or cytokine network.

Following on from this any agent which restores imbalance in the chemokine and/or cytokine network may be used in the manufacture of a medicament for use in the treatment of non-viral or non-autoimmune induced heart disorders or non-viral or non-autoimmune associated phases of heart disorders, or for use in the treatment or alleviation of symptoms associated with such heart disorders or phases. Any appropriate agent can be used in this regard, for example chemokine or cytokine antagonists or antibodies etc., however preferably the agent for use in this regard is IVIG. Preferably the disorder which is treated is CHF.

The above described uses are generally carried out on mammals. Any mammal may be treated with the above uses, for example humans, mice, rats, pigs, cats, dogs, sheep, rabbits, horses, cows or monkeys. However, preferably the mammals are humans.

As described above patients with heart disorders such as those defined herein, i.e. non-viral or non-autoimmune induced heart disorders, and in particular CHF, can exhibit a general up-regulation in levels of pro-inflammatory chemokines, cytokines and their receptors together with a general down regulation of anti-inflammatory chemokines, cytokines and their receptors.

Thus, a yet further aspect of the present invention provides a method of testing to see whether a patient with the heart disorders as defined herein, and in particular CHF, is a good candidate for treatment with immunomodulating agents and in particular IVIG, comprising analysing a patient's blood sample for up-regulation or elevated levels of pro-inflammatory chemokines, cytokines or their receptors and/or the down-regulation or reduction in levels of anti-inflammatory chemokines, cytokines or their receptors. Particular examples of cytokines, chemokines and their receptors which may be assayed for up-regulation or elevation are IL-1β, TNF-α, IL-1, MIP-1α, MIP-1β, IL-8, CCR1, CCR2, CCR5, CXCR1, CXCR2 and CX₃CR. Examples of cytokines, chemokines and their receptors which may be assayed for down-regulation or reduction are IL-10, IL-1Ra, sTNFRs, CCR7 and CXCR5. Patients which show an increase in one or more of the "pro-inflammatory" chemokines, cytokines and/or their receptors and/or a reduced level of one or more "anti-inflammatory" chemokines, cytokines and/or their receptors are good candidates for treatment with immunomodulating agents and in particular IVIG.

The above described "increase" or "elevation" or "up-regulation" in levels of chemokines, cytokines and their receptors or the above described "decrease" "reduction" or "down-regulation" in levels of chemokines, cytokines and their receptors refers to any measurable change in level of the entity in question when compared to its level in patients which do not display the symptoms of the particular heart failure in question. Preferably the "increase" "decrease" etc, i.e. the change in level of the entity in question will be statistically significant. Methods of determining statistical significance of parameter are well known in the art and described in more detail above.

For use according to the present invention, there may be provided a pharmaceutical composition, said composition comprising IVIG together with at least one pharmaceutically acceptable carrier, diluent or excipient.

The active ingredient in such compositions may comprise from 0.05% to 99% by weight of the formulation, more preferably from 0.1% to 1.0% and most preferably around 0.5%.

By "pharmaceutically acceptable" is meant that the ingredients must be compatible with other ingredients of the composition as well as physiologically acceptable to the recipient.

The pharmaceutical compositions may be formulated according to any of the conventional methods known in the art and widely described in the literature. Thus, the active ingredient may be incorporated, optionally together with other active substances, with one or more conventional carriers, diluents and/or excipients, to produce conventional galenic preparations which are suitable or can be made suitable for subcutaneous, intramuscular or intravenous administration such as powders, sachets, cachets, elixirs, suspensions, emulsions, solutions, syrups, ointments, sterile injectable solutions, sterile packaged powders, and the like. Preferably the pharmaceutical composition comprising the immunomodulating agent is prepared in a form appropriate for infusion or injection into a patient. Such infusion or injection is preferably intravenous (i.v.) but may also be given subcutaneously (s.c.) or intramuscularly (i.m.).

Suitable protocols for i.v., i.m. and s.c. administration are well known and standard in the art. An exemplary protocol might be slow intravenous infusion of IVIG at 0.75-1 mL/minute for 15 minutes, thereafter 1.2-1,5 mL/minute for 15 minutes, and thereafter 2.5 mL/minute throughout the infusion time.

Examples of suitable carriers, excipients, and diluents are lactose, dextrose, sucrose, maltose, glucose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, water/ glycol, water/polyethylene, glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, preserving agents, sweetening agents, flavouring agents, and the like. The compositions of the invention may be formulated so as to provide quick, sustained or delayed release of the active ingredient after administration to the patient by employing procedures well known in the art.

As described above, preferably the composition is in a form suitable for infusion or injection and preferred carriers are water and a carbohydrate substance such as maltose or glucose. Such carriers may be present at any appropriate concentration, but exemplary concentrations are from 1% to 20% and preferably from 5% to 10%.

Suitable doses will vary from patient to patient and can be determined by the physician in accordance with the weight, age and sex of the patient and the severity of the condition. Preferred doses of IVIG are from 0.4 g/kg to 2 g/kg.

A typical treatment may involve an induction therapy comprising an infusion of the immunmodulating composition into the patient each day for several days at the start of treatment and then infusions on a monthly basis for an appropriate number of months thereafter. The number of monthly infusions to give will be determined based on the patient's response to the treatment.

For example, a typical treatment may involve an induction therapy to infuse a total of 2 g/kg of active ingredient (e.g. IgG) into a patient over a number of days, for example 0.4 g/kg for each of 5 days. Thereafter the patients will typically receive an infusion each month (for example an infusion of 0.4 g/kg each month) for a total of 6 months, with the possibility of extending this treatment for a further number of months and even through the whole lifetime of a patient.

The improvements seen in patients treated in accordance with the present invention may be immediate (e.g. after a few days), or may be seen after a few weeks or a few months depending on the individual patient. Once the initial improvement is seen, continued improvement over the subsequent weeks and months may also occur. As indicated above, treatment can be continued for as long as is desired or is necessary.

Any of the procedures well known and documented in the art may be used to give the infusions to the patients, a preferred method being that described in "Felleskatalogen" (Felleskatalogen, 1999, volume 41, page 1081-2; Felleskatalogen A/S, Oslo, Norway. ISBN 82-90545-84-3). Alternatively, but less preferably, the immunomodulating agent could be infused or injected into the patient in one initial session and this could optionally be followed up with monthly infusions.

Use of IVIG as an immunomodulating agent in accordance with the present invention may be in place of or preferably in addition to the use of other drugs for treatment of heart disorders. Thus, other drugs known to treat heart disorders might be included in the pharmaceutical compositions described above or may be administered separately, in a manner appropriate for the drug concerned.

Thus, in a further aspect the present invention provides a product comprising (a) an immunomodulating agent being IVIG and (b) a second drug (e.g. a second agent effective for the treatment of heart disorders) as a combined preparation for simultaneous, separate or sequential use in the treatment of heart disorders or in improving cardiac function.

The heart disorders to be treated are as defined above. Suitable second drugs or agents are well known and documented in the art and include known drugs for use in the treatment of heart disorders, for example diuretics, vasodilators, inotropic drugs such as digoxin, or other compounds such as anticoagulants, β blockers, angiotensin II blockers or angiotensin converting enzyme (ACE) inhibitors may be used as discussed above.

The invention will be further described with reference to the following non-limiting Examples with reference to the following drawings in which:

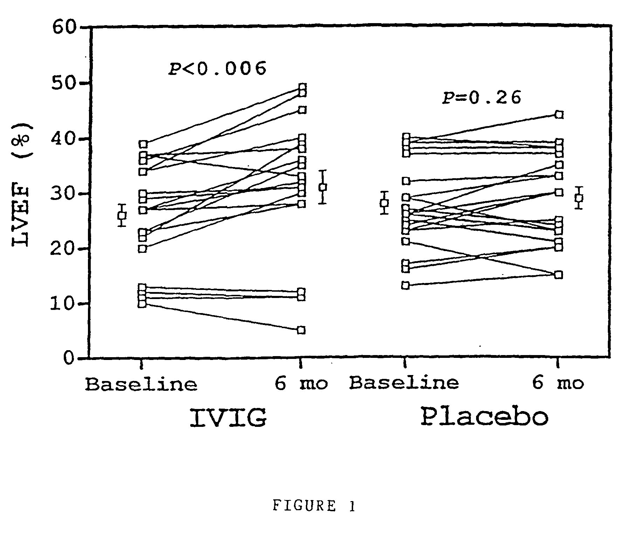

Figure 1 shows that LVEF (as assessed by ECG synchronized gated radionuclide ventriculography at rest) increased significantly after 6 months of IVIG treatment, whilst no significant change was seen in the placebo group. The mean ± SEM is indicated.
Figure 2 shows that plasma levels of Nt-proANP are decreased significantly over time during IVIG treatment whilst no significant change is seen in the placebo group. Data are given as mean ± SEM. * = p<0.05, ** = p<0.01 and *** = p<0.001 versus baseline. # = p<0.05 comparing differences in changes between the IVIG and placebo group.

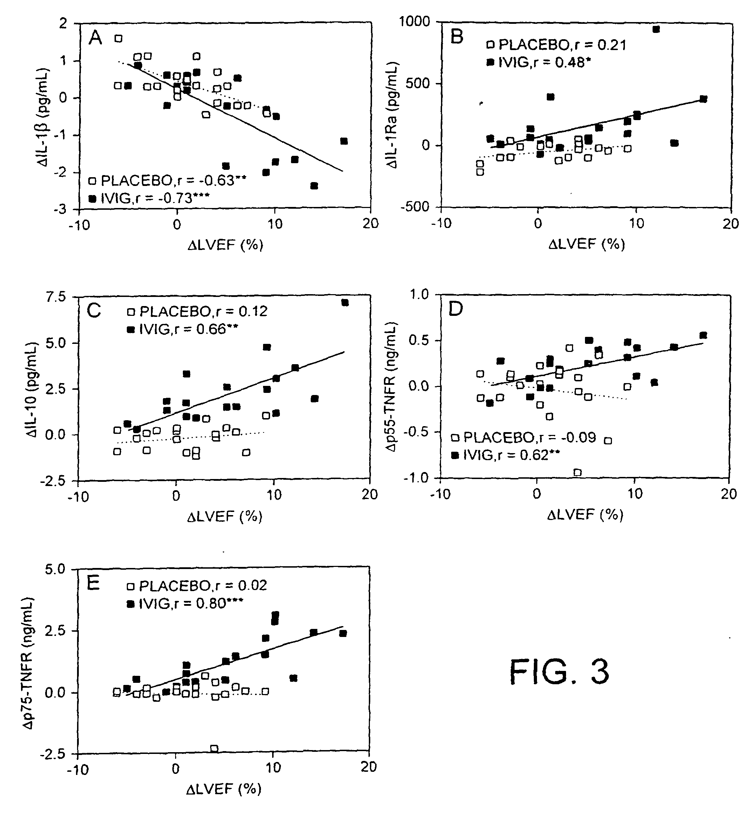

Figure 3 shows correlations between absolute change in left ventricular ejection fraction (LVEF%), as assessed by ECG synchronized gated radionuclide ventriculography at rest, and absolute change in plasma levels of IL-1β(A), IL-1Ra(B), IL-10(C), soluble p55-TNFR(D) and soluble p74-TNFR(E) in 39 CHF patients receiving IVIG (n=19) or placebo (n=20) for 6 months. * = p<0.05, ** = p<0.01 and *** = p<0.001.

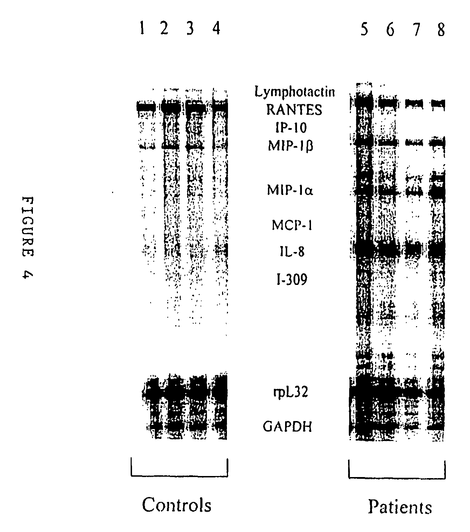

Figure 4 shows RNase protection assay (RPA) analysis of chemokine gene expression in mononuclear cells (MNC). Representative RNase protection assays (RPA) of chemokine gene expression in MNC from mixed venous blood (pulmonary artery) in 4 CHF patients and peripheral blood in 4 healthy controls are shown.

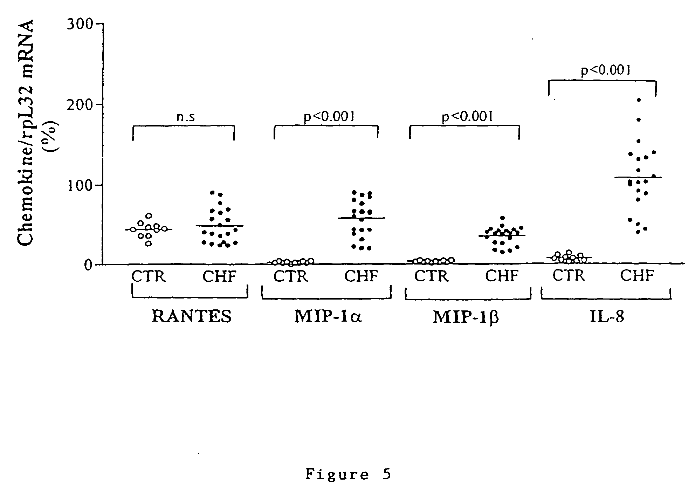

Figure 5 shows chemokine gene expression in MNC from CHF patients and controls. Relative mRNA levels of RANTES, MIP-1α, MIP-1B and IL-8 in mononuclear cells (MNC) from mixed venous blood (pulmonary artery) in 20 CHF patients and peripheral blood in 10 healthy controls are shown. Horizontal lines indicate means of observations. Data are presented as percentage of rpL32 expression. MIP-1α, MIP-1B and IL8 show significantly increased expression in CHF patients whereas RANTES expression is essentially unchanged.

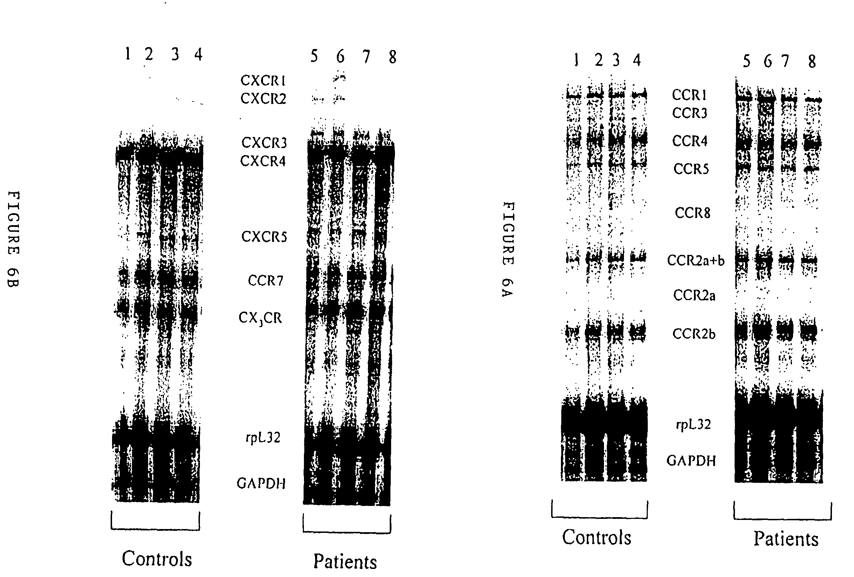

Figure 6 shows RPA analysis of chemokine receptor gene expression in MNC. Representative RNase protection assays (RPA) of CC-(A) and CXC-chemokine receptor (B) gene expression in mononuclear cells (MNC) from mixed venous blood (pulmonary artery) in 4 CHF patients and peripheral blood in 4 healthy controls are shown.

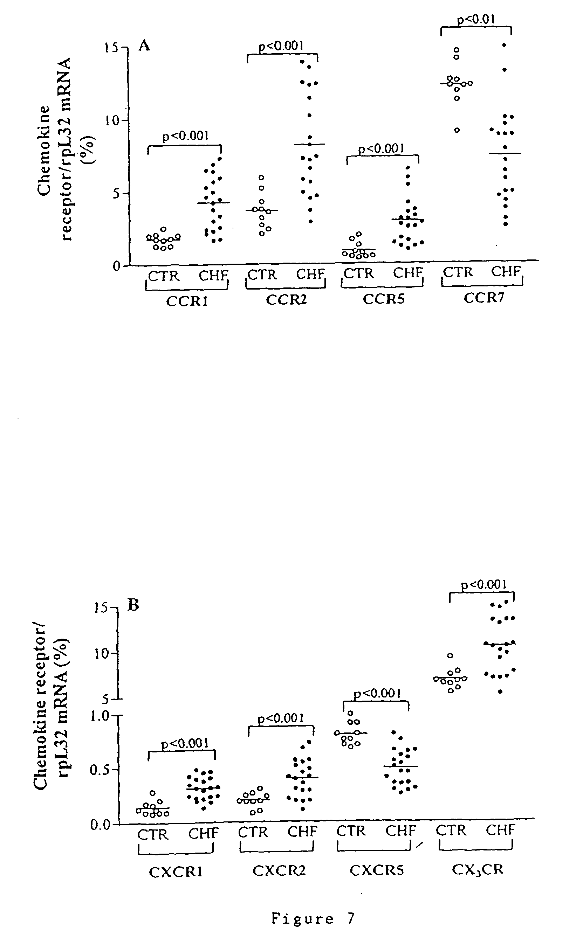

Figure 7 shows chemokine gene expression in MNC from CHF patients and controls. Relative mRNA levels of CC-(A) and CXC-chemokine receptor (B) in mononuclear cells (MNC) from mixed venous blood (pulmonary artery) in 20 CHF patients and peripheral blood in 10 healthy controls are shown. Horizontal lines indicate means of the observations. Data are presented as percentage of rpL32 expression. CCR1, CCR2, CCR5, CXCR1, CXCR2 and CX₃CR show significantly increased expression in CHF patients, whereas CXCR5 and CCR7 expression is significantly reduced in CHF patients.

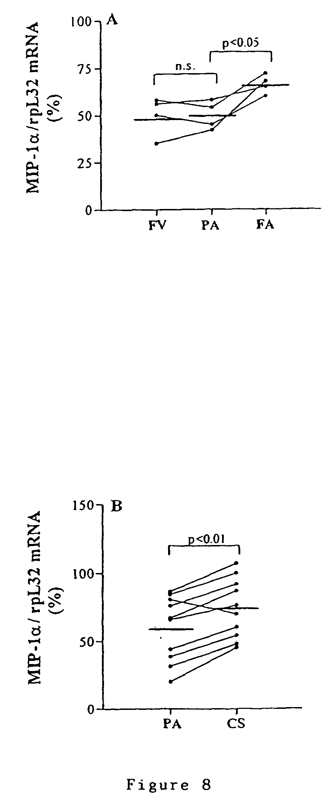

Figure 8 shows MIP-1α gene expression in MNC from different blood compartments. Relative mRNA levels of MIP-1α in mononuclear cells (NMC) from different blood compartments; femoral vein (FV), femoral artery (FA) and pulmonary artery (PA) in 4 CHF patients undergoing both left and right heart catheterization (A), and PA and coronary sinus (CS) in 10 CHF patients undergoing right-sided heart catheterization (B) are shown. Horizontal lines indicate means of the observations. Data are pressed as a percentage of rpL32 expression.

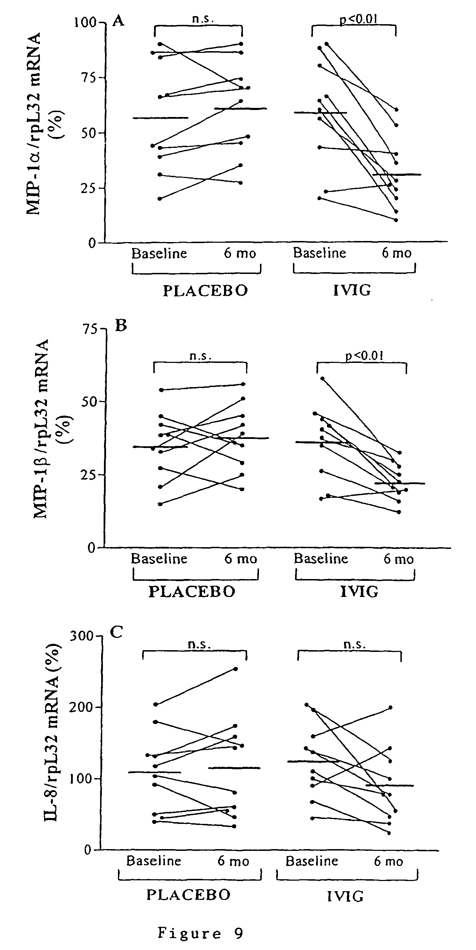

Figure 9 shows the effect of IVIg therapy on chemokine gene expression in MNC. Relative mRNA levels of MIP-1α(A), MIP-1B(B) and IL-8 (C) in mononuclear cells (MNC) from mixed venous blood (pulmonary artery) in 20 CHF patients before and after 6 months (mo) of treatment with IVIg (n=10) or placebo (n=10) are shown. The expression of MIP-1α(p<0.01) and MIP-1B(p<0.01) is significantly reduced in CHF patients receiving IVIg. Horizontal lines indicate means of the observations. Data are presented as a percentage of rpL32 expression.

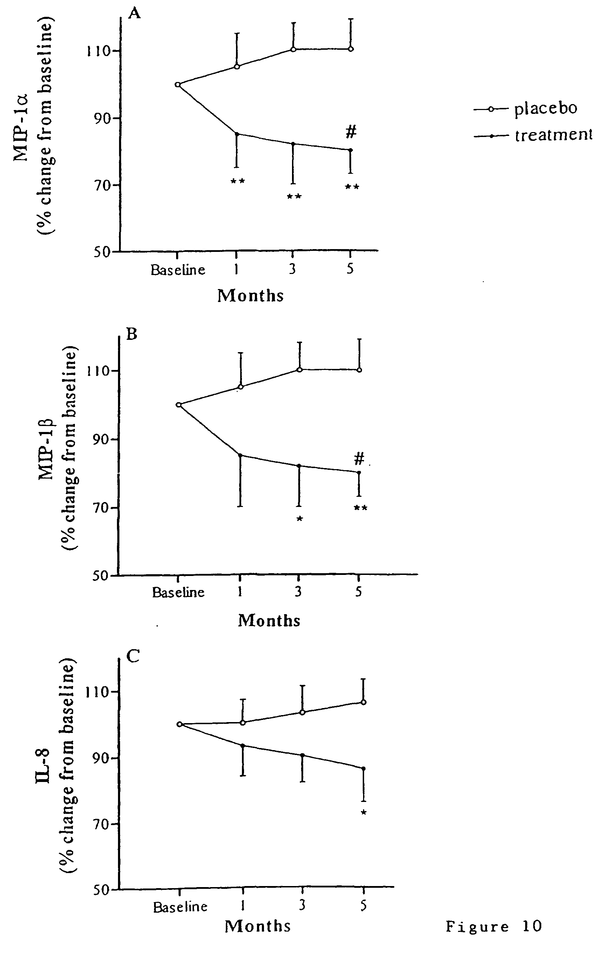

Figure 10 shows the effect of IVIg therapy on chemokine plasma levels. Plasma levels of MIP-1α(A), MIP-1B(B) and IL-8(C), given as a percentage change from baseline (MIP-1α:25.2±2.0 pg/ml, MIP-1B(B): 93.4±4.5 pg/ml, IL-8: 22.8±1.8 pg/ml), in 20 CHF patients before and at different time points after initiating treatment with IVIg (n=10) or placebo (n=10). Data are given as mean±SEM. *p<0.05, **p<0.01 versus baseline; #p<0.05 comparing changes between IVIG and the placebo group. Mo, months. It can be seen that expression of MIP-1α and MIP-1β, as well as the expression of IL-8 is significantly reduced in CHF patients receiving IVIG.

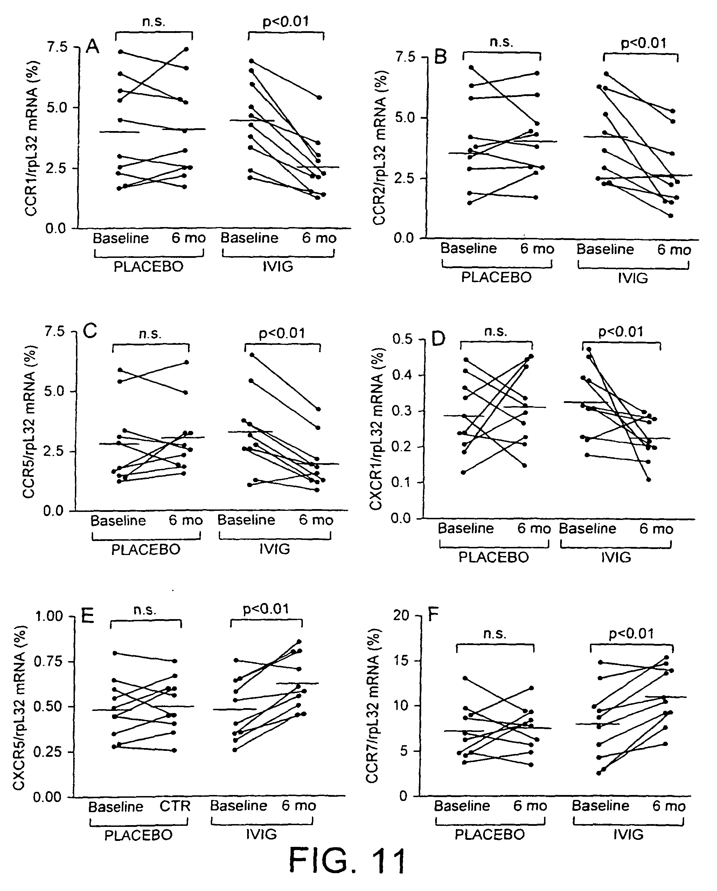

Figure 11 shows the effect of IVIg therapy on chemokine receptor gene expression in MNC. Relative mRNA levels of CCR1 (A), CCR2 (B), CCR5 (C), CXCR1 (D), CXCR5 (E) and CCR7 (F) in mononuclear cells (MNC) from the mixed venous blood (pulmonary artery) in 20 CHF patients before and after 6 months (mo) of treatment with IVIg (n=10) or placebo (n=10) are shown. The expression of CCR1 (p<0.001), CCR2 (p<0.01), CCR5 (p<0.01) and CXCR1 was significantly reduced in CHF patients receiving IVIG, whereas expression of CCR7 (p<0.05) and CXCR5 is significantly increased in CHF patients receiving IVIG. Horizontal lines indicate means of the observations. Data are presented as percentage of rpL32 expression.

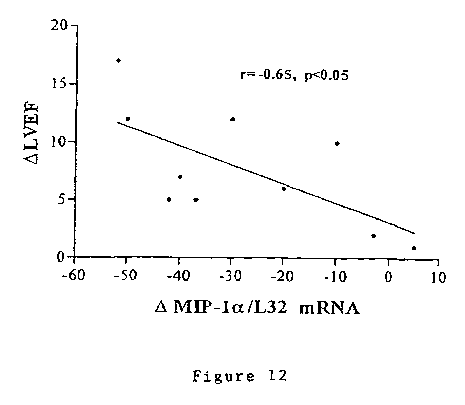

Figure 12 shows the correlation between change in LVEF and MIP-1α gene expression during IVIg therapy. Correlation between absolute change in left ventricular ejection fraction (LVEF%), as assessed by ECG synchronized gated radionuclide ventriculography at rest, and absolute change in mRNA levels of MIP-1α in 10 CHF patients receiving IVIG for 6 months (mo) is shown. Relative mRNA levels are presented as percentage of rpL32 expression.

### Example 1

### Clinical trial protocol - Methods

### Patients

Forty patients with chronic stable CHF for >6 months were included in the study (Table 1). The patients were included if they: (i) were classified in New York Heart Association (NYHA) functional class II to III, (ii) had left ventricular ejection fraction (LVEF) <40%, (iii) had no changes in medication during the last 3 months and (iv) were on optimal medical treatment and considered unsuitable for surgical intervention. Patients were not included if they had: (i) evidence of myocardial infarction or unstable angina during the last 6 months or (ii) significant concomitant diseases such as infections, pulmonary disease or connective tissue disease. The conventional cardiovascular treatment regimen was not changed during the study in any of the patients.

The etiology of CHF was classified as coronary artery disease (CAD, n=23) or idiopathic dilated cardiomyopathy IDCM, n=17) based on disease history and coronary angiographic examination. The Regional Ethical Committee approved the study. Signed informed consent was obtained from each patient.

### IVIG preparation

Octagam (Octapharma, Vienna, Austria), produced from fresh frozen plasma collected from Norwegian blood banks (Aukrust P., Frøland S.S., Liabakk N.K. et al., Release of cytokines, soluble cytokine receptors and interleukin-1 receptor antagonist after intravenous immunoglobulin administration in vivo., Blood 1994; 84: 2136-43) is dispensed in sterile water containing 10% maltose (final IgG concentration 5 g/L). Using the enzyme immunoassays (EIAs) described below, we could not detect IL-1β, IL-1 receptor antagonist (IL-1Ra), TNFα, soluble TNF receptors (sTNFRs) or IL-10 in the IVIG product. The endotoxin levels in the IVIG and placebo (see below) preparation were <10 pg/mL.

### Study design

After baseline measurements the patients were randomized to IVIG or placebo (5% glucose) in a double-blind fashion, and stratified according to etiology (i.e. CAD and IDCM). IVIG or an equal volume of placebo was given at a rate according to the manufacturer's instruction as induction therapy (one daily infusion (0.4 g/kg) for 5 days), and thereafter as monthly infusions (0.4 g/kg) for a total of 5 months. Baseline measurements were repeated at the end of study (26 weeks, 4 weeks after last IVIG or placebo infusion). One person not participating in any of the analytical procedures performed all IVIG and placebo administrations.

The primary end-point in the study was changes in LVEF. In addition we wanted to explore the effect of IVIG on predefined functional, hemodynamic, echocardiographic hormonal and immunologic variables.

### Methods

At baseline and at the end of study the following tests were performed: (i) LVEF was assessed by ECG synchronized gated radionuclide ventriculography at rest; (ii) hemodynamic variables were assessed by right-sided heart catheterization; (iii) echocardiographic variables were obtained by using a GE Vingmed System Five instrument and interpreted by a single cardiologist; (iv) exercise testing using an electrically braked bicycle ergometer. The test protocol consisted of a starting work rate of 20 W increasing by 20 W every second minutes until exhaustion (defined as an inability to keep the pedalling rate steady at 60 rpm). Oxygen uptake (VO₂) was measured using the EOS/SPRINT system. Peak VO₂, was taken as the highest VO₂ observed; (v) clinical evaluation was assessed by NYHA classification performed by a single cardiologist; (vi) plasma levels of N-terminal proatrial natriuretic peptide (Nt-proANP)(see below); (vii) immunologic variables (see below).

### Blood sampling protocol

Blood samples for cytokine analyses were collected into pyrogen-free vacuum blood collection tubes with EDTA as anticoagulant during right-sided heart catheterization from the pulmonary artery (mixed venous blood). The tubes were immediately immersed in melting ice, centrifuged within 15 minutes at 1000g and 4°C for 15 minutes and plasma was stored at -80°C until analysis. Samples were thawed only once. Plasma for measurements of hematologic parameters, clinical chemistry and Nt-proANP was collected from peripheral veins.

### Laboratory analyses

TNFα; and IL-10 were measured by EIAs (Biosource Internationale, Camarillo, CA). Soluble TNFRs, p55 and p75, were analyzed by EIAs as described (Liakbakk N.B., Sundan A., Lien E. et al., The release of p55 receptor from U937 cells studied by a new p55 immunoassay, J. Immunol Methods 1993; 163: 145-54). IL-1Ra and IL-1β were examined by EIA obtained from R&D Systems, Minneapolis, MN. The coefficients of variation were <10% for all EIAs. Routine clinical chemistry was analyzed as previously reported (Aukrust P., Berge R.K. Muller F., Ueland P.M., Svardal A.M., Frøland S.S, Elevated levels of reduced homocystein in common variable immunodeficiency-a marker of enhanced oxidative stress. Eur J. Clin. Invest 1997; 27:731-2). N-terminal proatrial natriuretic peptide (Nt-proANP) was measured by radioimmunoassay.

### Statistical analyses

We estimated the sample size required to be 40 patients for a two-tailed significance level <0.05 and a power of 0.80 to detect an increase in LVEF of 6%.

Differences between groups were compared with Student t test or Mann-Whitney U Rank-Sum test for unpaired data. In the paired situation, multiple analyses of variance (MANOVA) were performed a priori, and if significant, Wilcoxon's Rank-Sum test for paired data was performed. Relations between variables were tested using Spearman's Rank-Correlation test. The results are given as mean ± SEM, while the changes in variables after treatment are expressed as mean with associated 95% confidence interval (95% CI) if not otherwise stated. P values are two-sided and taken as significant when <0.05.

### Example 2

### Results

Key demographic and hemodynamic parameters were similar in the two treatment groups (Table 1). One patient in the IVIG group was withdrawn by local physician 3 weeks after baseline due to paroxysmal atrial fibrillation.

### Ventricular Function

LVEF as the primary end-point of the study, increased significantly by 5 EF units after IVIG, while no significant change was seen in the placebo group (Fig 1, Table 2). The change from baseline was also different between the IVIG and placebo group, although not reaching statistical significance (Table 2). No beneficial effect of IVIG was seen in the 4 patients with the lowest LVEF (<15% at baseline), while all but one of the other patients receiving IVIG had an increase in LVEF after therapy (Fig. 1). The effect of IVIG on LVEF was independent of etiology, increasing from 25 ± 3% to 30 ± 4% (p<0.05) in the CAD and from 26 ± 6% to 31 ± 4% (p<0.05) in the IDCM group, with no changes in the placebo group in either of these etiologic groups.
Right ventricular ejection fraction also improved after IVIG by an average of 6 EF units (p<0.05), with no significant change in the placebo group (Table 2).

### Hemodynamic and echocardiographic variables and exercise capacity

In those treated with IVIG hemodynamic variables obtained during right-sided heart catheterization showed a significant decrease in pulmonary capillary wedge pressure by -22%, and in pulmonary artery pressure, while no significant changes were observed in the placebo group (Table 2).

Technical satisfactory echocardiographic data were obtained in 30 patients (15 in the IVIG and 15 in the placebo group), and showed in general unchanged M-mode left ventricular dimensions, interventricular and posterior wall thickness and grade of mitral regurgitation in both groups (Table 2). However, mitral velocity deceleration time (DT) increased during IVIG and decreased during placebo treatment resulting in a significant difference between the groups (Table 2). After IVIG therapy exercise capacity as assessed by peak VO₂ increased by 6% and peak work load increased by 10%, while no changes were observed in the placebo group (Table 2). As for the peak work load, there was a significant difference between IVIG and placebo treatment (Table 2). Moreover, IVIG therapy in contrast to placebo, resulted in an increase in peak heart rate and peak systolic blood pressure (Table 2).

### Functional status

Functional status as assessed by NYHA class improved in the placebo and particularly in the IVIG group (Table 2).

### N-terminal proatrial natriuretic peptide (Nt-proANP)

Plasma levels of Nt-proANP, which have been found to correlate with pulmonary artery pressures in CHF, providing important prognostic information in CHF patients (Gottlieb S.S., Kukin K.L., Ahern D., Packer M., Prognostic importance of atrial natriuretic peptide in patients with chronic heart failure. J. Am. Coll. Cardiol 1989; 13: 1534-9) decreased significantly after the induction therapy and continued to decrease towards the end of the study during IVIG therapy (Fig. 2). No significant change was observed in the placebo group resulting in a significant difference between the two groups (Table 2, Fig. 2).

### Immunologic parameters

IL-1β increased significantly in the placebo, but not in the IVIG group (Table 3). Simultaneously, IVIG, but not placebo, induced a marked increase in IL-1Ra levels ^{~}57%) accompanied by a marked increase in IL-10 levels (^{~}65%) (Table 3). Finally, while TNFα tended to decrease after IVIG and increase after placebo treatment, IVIG, but not placebo induced a rise in both soluble p55 (^{~}15%) and particularly in soluble p75-TNFR (^{~}65%)(Table 3). Thus, it appears that IVIG had a profound anti-inflammatory effect in CHF as reflected in enhanced levels of IL-10, IL-1Ra and sTNFRs as well as decreased levels of IL-1β/1L-1Ra and TNFα/sTNFRs ratios. In contrast, no or even opposite changes were seen after placebo treatment, resulting in significant differences between the two groups for several of these parameters (Table 3). Notably, in the IVIG, but not in the placebo group, there was a strong positive correlation between the changes in IL-1Ra, IL-10 and both types of sTNFRs and the change in LVEF (Fig. 3). In contrast, the change in IL-1β was negatively correlated with the change in LVEF in both the IVIG and placebo group (Fig. 3).

### Side effects and laboratory status

Eight subjects (2 with placebo and 6 with IVIG) felt mild discomfort (chills or headache) during the first hours after infusion, 3 patients (all with IVIG) had a rise in temperature (<39.0°C) immediately after infusion, persisting for a few hours, and 3 patients (all with IVIG) developed a mild itching rash after infusion, persisting for a few days. None of the patients had to withdraw from the study because of these side effects. Blood was collected every month, but no changes in routine hematologic parameters, serum creatinine, ASAT. ALAT, electrolytes or glucose took place during the study. With this respect, no difference between the groups were observed.

### Discussion

The novel and important finding of the present prospective trial with IVIG in patients with CHF was improvement of LVEF, significantly correlated with an anti-inflammatory effect on the balance between inflammatory and anti-inflammatory mediators. The present double-blind, placebo-controlled study shows for the first time that immunomodulation can improve important parameters reflecting functional capacity, cardiac performance and hemodynamic status in patients with moderate to severe heart failure. Notably, this effect of IVIG was achieved in patients on optimal conventional cardiovascular treatment regimens including β-blockers. The improvement of cardiac performance was found in both IDCM and in ischemic cardiomyopathy. The findings demonstrate a potential for immunomodulating therapy in the treatment of CHF.

Although the effect may seem modest, the beneficial effects of IVIG are similar or even greater than those reported in controlled trials examining standard cardiovascular medications. For example, the increase in LVEF by 5 EF% is similar to what can be expected from β-blockers (Kukin M.L., Kalman J., Charney R.H. et al., Prospective, randomized comparison of effect of long-term treatment with metoprolol or carvedilol on symptoms, exercise, ejection fraction, and oxidative stress in heart failure. Circulation 1999; 99:2645-51) and greater than reported for ACE-inhibitors or digoxin (Captopril Multisenter Research Group. A placebo-controlled trial of captopril in refractory chronic congestive heart failure. J. Am. Coll. Cardiol 1983; 2: 755-63). Moreover, the improvement in peak VO₂, by 0.7 ml/kg/min is similar to the effect of combined treatment with isorbid-dinitrate and hydralazine or treatment with β-blockers and greater than the effects of ACE-inhibitors or calcium antagonists (Cohn J.N., Johnson G., Ziesche S. et al. A comparison of enalapril with hydralazine-isosorbide dinitrate in the treatment of chronic congestive heart failure. N. Engl.J. Med. 1991;325:303-10; Cohn J.N., Ziesche S., Smith R. et al. Effect of the calcium antagonist felodipine as supplementary vasodilator therapy in patients with chronic heart failure treated with enalapril. V-HeFT III. Circulation 1997;96:856-63).

Since an improvement in LVEF has been found to be associated with improved survival among CHF patients (Cintron G., Johnson G., Francis G., Cobb F., Cohn J.N. Prognostic significance of serial changes in left ventricular ejection fraction in patients with congestive heart failure. Circulation 1993;87 (suppl. VI): VI-17-VI-23) the primary goal was to examine the effect of IVIG on LVEF. However, the data suggest an overall beneficial effect of IVIG in CHF, not only on LVEF, but also on functional status, exercise capacity and hemodynamic variables. Moreover, comparing with placebo treatment, IVIG treatment reduced the invasive PCW pressure and increased the noninvasive DT parameter, possibly reflecting improved diastolic function. Although the study was not designed to examine changes in myocardial function throughout the study period, it was found that IVIG was associated with a decline in Nt-ANP after induction therapy, possibly reflecting improved hemodynamic status. Also, the decline in Nt-ANP was most pronounced at the end of the study, suggesting that the effect of IVIG might be even greater during longer follow-up. Furthermore, while the effect of IVIG on LVEF was independent of the etiology for CHF, it seems at least partly to depend on the degree of left ventricular dysfunction. Thus, patients with particularly low LVEF had no beneficial effect of IVIG, possibly reflecting a state of severe myocardial damage inaccessible for immunomodulation. Finally, although some patients experienced mild side effects, IVIG treatment was well tolerated and did not require discontinuation of therapy.

A major finding in the present study was that the effect of IVIG on LVEF was correlated with a marked rise in IL-10, IL-1Ra and sTNFRs levels, resulting in a combined enhancement of several anti-inflammatory mediators. Whilst not wishing to be bound by theory, several modes of action may explain the beneficial effect of IVIG in immune-mediated disorders (Dwyer J.M., Manipulating the immune system with immune globulin. New Engl. J. Med. 1992; 326:107-16), and some of these may be operative in CHF. Specifically, IVIG has been found to induce complement inactivation, impair Fas mediated apoptosis and inhibit leukocyte adhesion to endothelium. All these factors may be involved in the pathogenesis of CHF. However, our demonstration of a marked IVIG induced modulation of the cytokine network in anti-inflammatory direction, significantly correlated with increased LVEF, suggests that these effects may be of importance for the improved cardiac performance after IVIG treatment in CHF.

Based on the possible involvement of autoimmune or viral induced mechanisms, previous studies in humans and animal models of IVIG in heart disease have focused on myocarditis and new-onset cardiomyopathies. However, we found improved LVEF in both idiopathic dilated and ischemic cardiomyopathy. Whatever the initial mechanisms, the present study suggests that immune-mediated mechanisms may be part of a common final pathway operating in CHF independent of the etiology of heart failure, and that the mechanisms involved in this pathway at least partly may be down-regulated by IVIG.

Our findings indicate that IVIG improves functional capacity, cardiac performance and hemodynamic status in CHF patients, suggesting a potential for immunomodulating therapy in addition to optimal conventional cardiovascular treatment regimens in these patients.

### Example 3: Elucidation of the possible role of chemokines in the pathogenesis of CHF

### Patients

Mononuclear cells (MNC) were collected from twenty patients participating in the IVIg-study (see Table 4). Patients were included in the IVIg-study if they: (i) had chronic stable CHF for >6 months, (ii) were classified in New York Heart Association (NYHA) functional class II-III, (iii) had LVEF <40%, (iv) had no changes in medication during the last 3 months and (v) were on optical medical treatment and considered unsuitable for surgical intervention. Patients were not included if they had: (i) evidence of myocardial infarction or unstable angina during the last 6 months or (ii) significant concomitant disease such as infections, pulmonary disease or connective tissue disease. The conventional cardiovascular treatment was not changed during the study in any of the patients. The etiology of CHF was classified as coronary artery disease (CAD, n=10) or IDCM (n=10) based on disease history and coronary angiographic examination. For comparison, blood samples were also collected from ten healthy sex and age-matched controls. The study was approved by the Regional Ethics Committee and conformed with the Declaration of Helsinki. Signed informed consent was obtained from each patient.

### IVIG preparation

Octagam (Octapharma, Vienna, Austria), produced from fresh frozen plasma collected from Norwegian blood banks, was dispensed in sterile water containing 10% maltose (final IgG concentration 5g/L). Endotoxin levels in the IVIG and placebo (see below) were <10 pg/ml.

### Design of the IVIG study

After baseline measurements the patients were randomized to IVIG or placebo (5% glucose) in a double-blind fashion, and stratified according to etiology (i.e. CAD and IDCM). IVIG or an equal volume of placebo was given at a rate according to the manufacturer's instruction as induction therapy (one daily infusion (0.4 g/kg) for 5 days), and thereafter as monthly infusions (0.4 g/kg) for a total of 5 months. Baseline measurements were repeated at the end of study (26 weeks, 4 weeks after last IVIG or placebo infusion). One person not participating in any of the analytical procedures performed all IVIG and placebo administrations.

At baseline and at the end of the study LVEF was assessed by ECG synchronized gated radionuclide ventriculography at rest and blood samples from pulmonary artery (PA) and the coronary sinus (CS) were collected during right-sided heart catheterization. Plasma samples from peripheral venous blood were obtained at baseline and after 1, 3 and 5 months. In addition 4 CHF patients (not included in the IVIg-study) and 1 patient without heart failure (electrophysiological examination) underwent left- and right-sided heart catheterization to examine differences in chemokine gene expression: (I) between the femoral vein (FV), femoral artery (FA) and PA and (ii) in FV between start and end of the catheterization procedure.

### Collection of plasma

Blood samples for plasma were collected into pyrogen-free EDTA tubes, immediately immersed in melting ice, centrifuged within 15 minutes at 1000g for 15 minutes, and stored at -80°C until analysis. Samples were thawed only once.

### RNA preparation from MNC

MNC were obtained from heparinized blood by Isopaque-Ficoll (Lymphoprep, Nycomed Pharma AS, Oslo, Norway) gradient centrifugation within 45 minutes. Immediately after isolation, the suspension of MNC were centrifuged at 1000g for 10 minutes and the pellets were stored in liquid nitrogen. Total RNA was extracted from frozen cells using RNeasy columns (QIAGEN, Hilden, Germany) and stored in RNA storage solution (Ambion, Austin, Tx) at - 80°C until used. The integrity of the extracted total RNA concentration and purity was assessed by agarose gel electrophoreses and ethidium bromide staining. RNA concentration and purity was evaluated by absorbency at 260/280 nm by use of a spectrophotometer (GeneQuant; Pharmacia, Uppsala, Sweden).

### RNase protection assay (RPA)

RPA was used for the detection and quantification of mRNA species. Multi-probes hCK5 (1 C-, 5 CC-, and 2 CXC-chemokine probes), hCR5 (8 CC-chemokine receptor probes) and hCR6 (1 CC-, 5 CXC-, and 1CX₃C-chemokine receptor probes) were available with reagents for in vitro transcription and RPA (RiboQuant; Pharmingen, San Diego, CA). The following anti-sense RNA probes, able to hybridize with target human mRNA were synthesized: lymphotactin (Ltn), regulated on activation normally T-cell expressed and secreted (RANTES), interferon γ-inducible protein (IP)-10, macrophage inflammatory protein (MIP)-1α and -1β, monocyte chemoattractant protein (MCP)-1, interleukin (IL)-8 and inducible (I)-309, the CC-chemokine receptors (CCR)1-5, 7, 8, the CXC-chemokine receptors (CXCR)1-5, the CX₃C (fractalkine) receptor (CX₃CR) and ribosomal protein (rp) L32 and glyceraldehyd-3-phosphate dehydrogenase (GAPDH).

For all hybridization assays, approximately 2µg total RNA from each sample was mixed with 0.5-2x10⁶ cpm probe (α-³²P-UTP, 3000 Ci/mmol). Protected fragments were separated in a denaturing 6% polyacrylamide gel for 90 minutes. The dried gel was exposed to a phosphorimaging screen (Cyclone system; Packard, Meriden, CT) for 20 hours followed by densitometric analysis using ID Quantifier (Phoretix, Newcastle, UK). The mRNA signal was normalized to the signal obtained from rpL32 and GAPDH genes. The intra-experimental coefficient of variation (CV) was 12.2% (rpL32, n=20) and 16.4% (GAPDH, n=20).

### Miscellaneous

Plasma levels of MIP-1α, MIP-1β and IL-8 were measured by enzyme immunoassays (EIAs) (R&D Systems, Minneapolis, MN). The intra- and interassay CVs were <10% for all EIAs. The numbers of CD2⁺, CD4⁺, CD8⁺ and CD19⁺ lymphocytes and monocytes (CD14⁺ cells) in blood samples were determined by immunomagnetic quantification (23).

### Statistical analysis

Differences between groups were compared with Mann-Whitney Rank-Sum test for unpaired data. In the paired situation, Wilcoxon's signed-rank test for paired data was performed. Correlations between variables were tested using Spearman's Rank test. P values are two-sided and considered significant when <0.05.

### Example 4 - Results of the analysis of the role of chemokines in CHF

### Changes in MNC chemokine gene expression in human CHF

RPA as described in Example 3 documented substantial MNC chemokine gene expression in CHF patients and healthy controls. Out of the 8 chemokine genes tested, 7 were detected in CHF patients and controls (Figure 4). The gene expressions of MIP-1α (^{~}21 fold), MIP-1β (^{~}10-fold) and IL-8 (^{~}36-fold) were markedly increased in the patients compared to the controls (Figure 5). In contrast, RANTES was equally expressed in both groups (Figure 5). Ltn, MCP-1 and I-309 were expressed in both CHF patients and controls (Figure 4), but at very low levels. IP-10 was not detected in any sample.

### Changes in MNC chemokine receptor gene expression in human CHF

Out of the 13 chemokine receptor genes tested, 12 representing both CC-, CXC- and CX₃C-chemokine receptors were detected in both CHF patients and controls (Figure 6). Thus, concomitant with the enhanced expression of chemokines, the gene expression of several CCR (CCR1, CCR2 and CCR5), CXCR (CXCR1, CXCR2) as well as the fractalkine receptor CX₃CR were also significantly increased in MNC from CHF patients (figure 7). In contrast, the gene expression of CXCR5 and CCR7 were significantly reduced in the patients compared to the controls (Figure 7). No differences were found for CCR4 and CXCR4 (Figure 6). Gene expression of CCR3 and CXCR3 were detected in both patients and controls, although at very low levels (Figure 6). CCR8 mRNA was not detected in any samples. Except for a significantly higher expression of CXCR1 in CAD patients (p<0.05), the expression of chemokine and chemokine receptor genes did not differ between patients with ischemic and idiopathic dilated cardiomyopathy (data not shown).

### Chemokine and chemokine receptor gene expression in MNC isolated from different blood compartments

The differences between CHF patients and controls as described above could possibly reflect differences in gene expression between peripheral (controls) and central (CHF patients) venous blood or activation during right-sided heart catheterization. We therefore compared chemokine and chemokine receptor gene expression in MNC isolated from FV and PA, and the expression of these genes before and after catherization, in 4 CHF patients and 1 patient without myocardial failure (see methods in Example 3). Very similar mRNA levels of all chemokine and chemokine receptor genes were detected in FV and PA (Figure 8), and there was no significant induction of chemokine or chemokine receptor mRNA during the catheterization procedure in these patients (data not shown). Moreover, there were no differences in numbers of CD2⁺, CD4⁺, CD8⁺ and CD19⁺ lymphocytes or monocytes (CD14⁺ cells) between CHF patients (PA) and controls (peripheral venous blood) (data not shown). It is therefore unlikely that the reported differences in gene expression between patients and controls should merely reflect differences in the relative proportion of MNC subsets.

To further examine the chemokine system in CHF, we compared the gene expression of chemokines and their receptors in MNC isolated from peripheral venous (FV), mixed venous (PA) and arterial (FA) blood in 4 CHF patients not participating in the IVIg-study (see methods in Example 3) and between PA and blood from CS in 10 patients of the "IVIg-population". The mRNA level of MIP-1α was significantly higher in MNC from arterial blood (FA) compared to cells from venous blood (FV and PA) (Figure 8A). Moreover, gene expression of this chemokine was also significantly increased in cells from CS compared to PA (Figure 88). Although not significant, similar patterns of chemokine gene expression in different blood compartments were found for MIP-1 and IL-8.

### Effect of IVIg therapy on chemokine and chemokine receptor gene expression

Our results document that MNC from CHF patients are characterized by marked alterations in gene expression of both chemokines and the corresponding receptors. We next examined if IVIg could modulate expression of these genes in MNC isolated from central venous blood (PA) in 10 CHF patients receiving IVIg and 10 patients receiving placebo in a double blind fashion for 26 weeks (see methods in Example 3). As shown in Figure 9, the gene expression of MIP-1α and MIP-1β decreased during IVIg treatment, but not during placebo, and this decrease was seen in all but one patient. Also the mRNA levels of IL-8 tended to decrease (p=0.07). The gene expression of RANTES and other detectable chemokines was stable during both IVIg and placebo treatment (data not shown). Thus, the markedly enhanced gene expression of MIP-1α, MIP-1β and IL-8 in MNC from CHF patients seems to be down-regulated during IVIg therapy.

To further elucidate the effect of IVIg on chemokines, we measured plasma levels of MIP-1α, MIP-1β and IL-8 at a baseline and after 1, 3 and 5 months of therapy. While MIP-1α, MIP-1β and IL-8 tended to increase in the placebo group, they significantly decreased during IVIg therapy resulting in significant differences in changes between the groups for MIP-1α, MIP-1β. Moreover, in the IVIg group the levels of these chemokines decreased after induction therapy and continued to decrease throughout the study period (Figure 10).

Also the expression of chemokine receptors were influenced by IVIg. CCR1, CCR2, CCR5 and CXCR1 all decreased during IVIg treatment (Figure 11). In contrast, IVIg significantly increased the mRNA levels of CXCR5 and CCR7 in MNC (Figure 11), and as described above, the expression of these receptors were decreased in the CHF population before therapy (Figure 7). Neither IVIg nor placebo influenced the mRNA levels of other detectable chemokine receptors (data not shown).

In contrast to the changes in gene expression of chemokines and chemokine receptors, neither IVIg nor placebo induced any changes in the proportion of MNC subsets (i.e. CD2⁺, CD4⁺, CD8⁺ and CD19⁺ lymphocytes and monocytes (CD14⁺ cells)) (data not shown).

### Chemokine and chemokine receptor gene expression in MNC in relation to LVEF

The gene expression of MIP-1α, (r=-0.66, p<0.01), MIP-1β (r=-0.52, p<0.05), IL-8 (r=-0.40, p<0.07), CCR1 (r=-0.64, P<0.01) and CCR2 (R=-0.51, P<0.05) were inversely correlated with LVEF at baseline. We have shown in Example 2 a significant increase in LVEF (5EF units) after IVIg, but not after placebo treatment in these CHF patients (see Figure 1 and Table 2). Importantly, the decrease in MIP-1α mRNA levels during IVIg therapy was significantly correlated with the increase in LVEF after such therapy (Figure 12).

### Discussion

The study described in Examples 3 and 4 shows markedly altered gene expression of several chemokines and their corresponding receptors in MNC from CHF patients. Furthermore, the abnormal expression of chemokines and chemokine receptors in MNC from patients with CHF was significantly modulated in a normal direction during IVIg therapy, and for MIP-1α levels, the down-regulation was significantly correlated with an improved LVEF. These findings support a role for abnormal chemokine activation in the pathogenesis of CHF, and suggest that IVIg modulates such abnormal activity.

In the study increased gene expression of both CC- (i.e. MIP-1α and MIP-1β) and CXC-chemokines (i.e. IL-8) in MNC from CHF patients is shown, with particularly high expression of MIP-1α and MIP-1β in patients with low LVEF. It is also demonstrated that enhanced gene expression of several of the corresponding chemokine receptors in MNC from these patients occurs.

Gene expression of MIP-1α was higher in FA compared to FV and PA, and in CS compared to PA. These observations may indicate an activation of chemokine expression in MNC during passage through the lung and heart circulation, respectively. There are several potential stimuli for this "locally" increase chemokine expression in CHF patients, including hypoxia and increased coronary shear stress. However, migration of MNC into hypoxic and inflamed peripheral tissue may also decrease the amount of chemokine expressing MNC in venous blood. Nevertheless, increased gene expression of MIP-1α in CS may suggest enhanced activation of this gene in the failing myocardium.

In contrast to increased expression of CCR1, CCR2, CCR5, CXCR1 and CXCR2, we found that gene expression of the chemokine receptors CXCR5 and CCR7 were significantly reduced in CHF patients. Interestingly, we found that IVIg induced increased gene expression of both CXCR5 and CCR7 in MNC of CHF patients. Recent reports indicate that these receptors may be important mediators of constitutive homing of lymphocytes from the blood into secondary lymphoid organs (Forster et al; 1999, Cell., 99: 23-33 and Legler et al., 1998, J. Exp. Med., 187: 655-660). Thus, whilst not wishing to be bound by theory, it is possible that IVIG induces improved lymphocyte homing in CHF patients as part of its mechanism of action.

The present study is the first to demonstrate markedly altered gene expression of chemokine and chemokine receptor genes in MNC from CHF patients. The correlation between enhanced expression of both MIP-1α and MIP-1β and their corresponding receptor genes and depressed LVEF in these patients supports a role for chemokines in the pathogenesis of CHF. Of even more importance, our findings suggest that IVIg may down-regulate increased gene expression of the "inflammatory" chemokines and their receptors, while the expression of chemokine receptor genes involved in constitutive lymphocyte homing may be enhanced during such therapy. Thus, IVIg therapy may represent a novel therapeutical approach with the potential to restore a disbalance in the chemokine network in cardiovascular and possibly also other inflammatory diseases.

**Table 1.**

| Clinical characteristics of the CHF patients participating in the study | | |
|---|---|---|
| | IVIG n=20 | Placebo n=20 |
| Age (years) | 60 ± 1.3 | 61 ± 2.0 |
| Gender (males/females) | 18/2 | 15/5 |
| Body mass index (kg/m²) | 26.5 ± 0.8 | 26.1 ± 0.8 |
| Cause of heart failure | | |
| CAD | 11 (55%) | 12 (60%) |
| IDCM | 9 (45%) | 8 (40%) |
| Duration of heart failure (years) | 3.7 ± 0.5 | 3.4 ± 0.4 |
| Left ventricular ejection fraction (%) | 26 ± 2 | 29 ± 2 |
| Right ventricular ejection fraction (%) | 29 ± 3 | 32 ± 2 |
| Peak VO₂ (ml/kg/min) | 14.7 ± 0.7 | 14.1 ± 0.9 |
| Medication (%) | | |
| ACE inhibitors | 19 (95%) | 18 (90%) |
| Angiotensin-II blockers | 1(5%) | 2(10%) |
| Diuretics | 17 (85%) | 19 (95%) |
| β-blockers | 17 (85%) | 13 (65%) |
| Digitoxin | 7 (35%) | 9 (45%) |
| CAD, coronary artery disease; IDCM, idiopathic dilated cardiomyopathy: ACE. angiotensin converting enzyme. Data are given as mean ± SEM. | | |

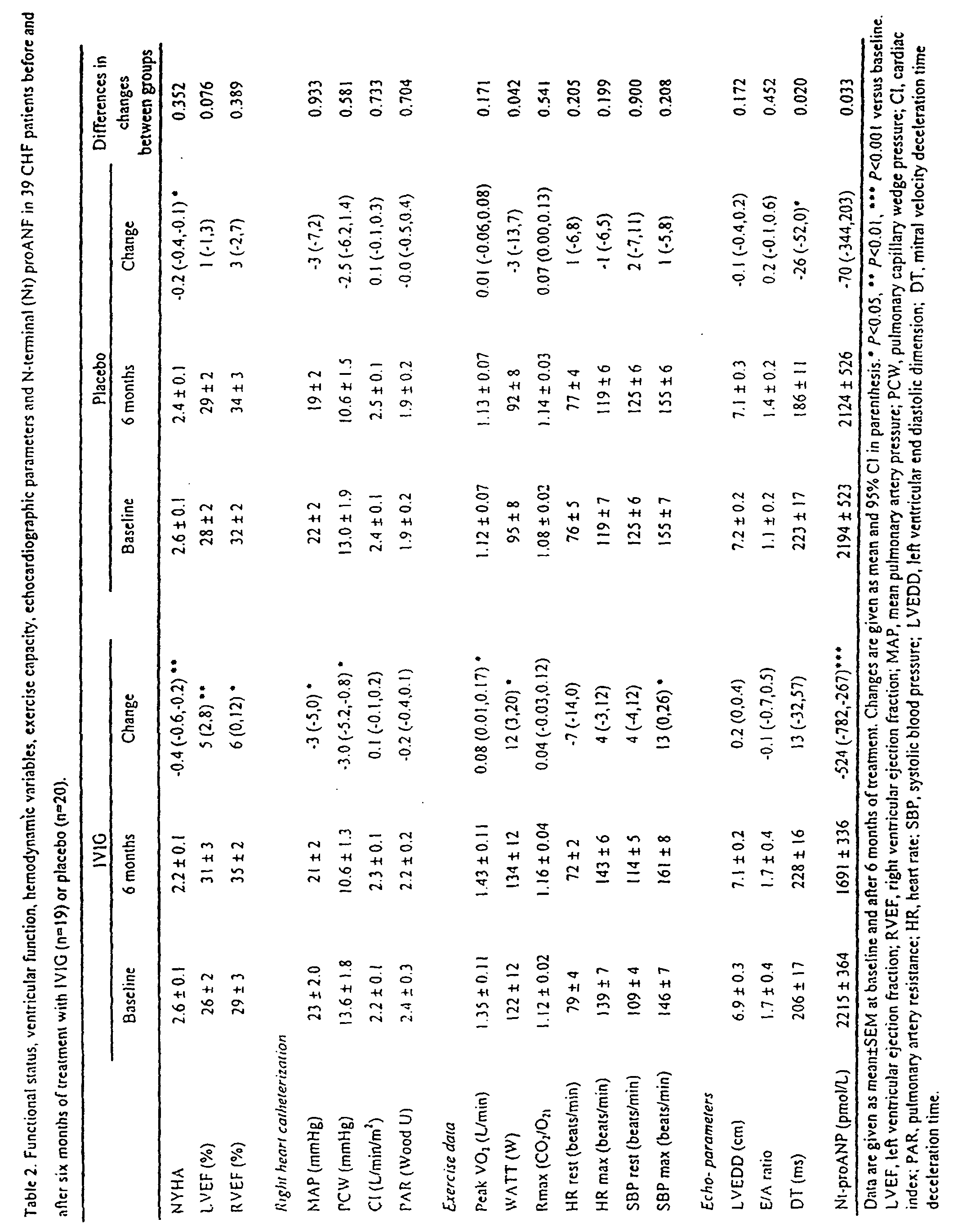

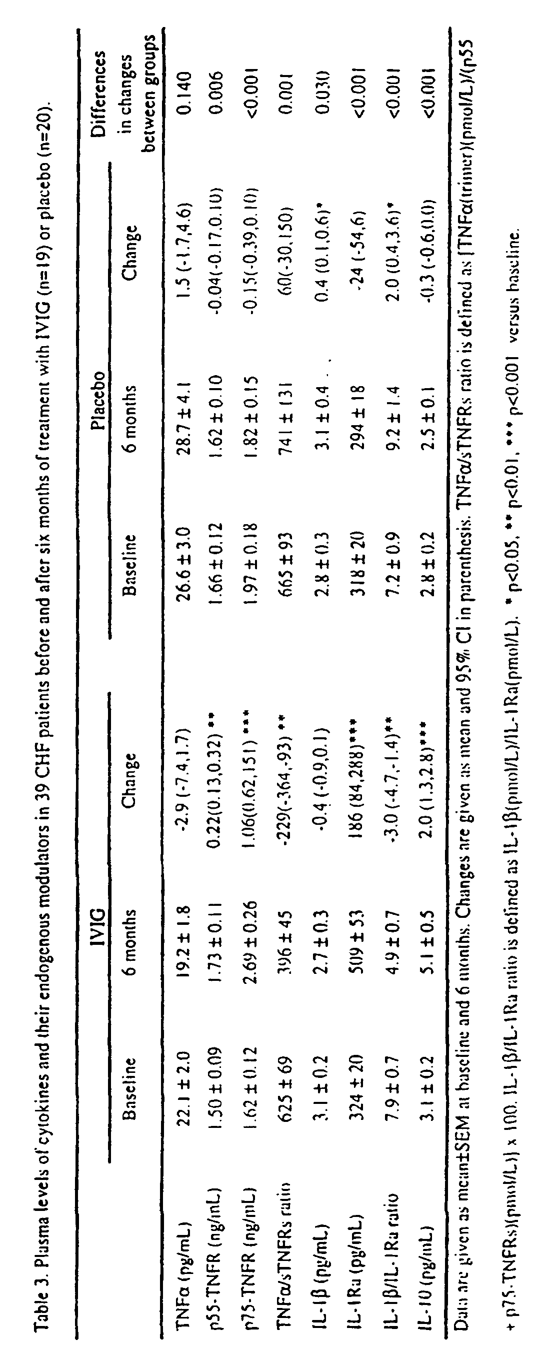

**Table 4.**

| **Clinical characteristics of the CHF patients participating in the study** | | |
|---|---|---|
| | **IVIG (n=10)** | **Placebo (n=10)** |
| Age (years) | 59±1.6 | 61±2.2 |
| Gender (male/female) | 8/2 | 7/3 |
| NYHA functional class (II/III) | 4/6 | 3/7 |
| Cause of heart failure | | |
| CAD | 5 | 5 |
| IDCM | 5 | 5 |
| Duration of heart failure (years) | 3.6±0.8 | 3.3±0.7 |
| Left ventricular ejection fraction (%) | 25±2 | 27±3 |
| Medication | | |
| ACE-inhibitors | 10 | 9 |
| Angiotensin-II blockers | 0 | 1 |
| Diuretics | 9 | 10 |
| β-blockers | 8 | 7 |
| Digitalis | 4 | 5 |
| CAD, coronary artery disease; IDCM, idiopathic dilated cardiomyopathy; ACE, angiotensin converting enzyme. Data are given as mean ± SEM | | |

## Claims

1. Use of intravenous immunoglobulin (IVIG) in the manufacture of a medicament for use in the treatment of non-viral or non-autoimmune induced heart disorders or non-viral or non-autoimmune associated phases of heart disorders, wherein said heart disorder is an ischaemic heart disease or a chronic non-ischaemic cardiomyopathy.

2. Use of intravenous immunoglobulin (IVIG) in the manufacture of a medicament for use in the treatment or alleviation of symptoms associated with the heart disorders or phases thereof as defined in claim 1.

3. Use as claimed in claim 1 or claim 2 wherein the heart disorder to be treated is selected from the group comprising chronic CHF, chronic (stable) angina pectoris and acute coronary syndromes.

4. Use as claimed in any one of claims 1 to 3, wherein said heart disorder is chronic ischaemic cardiomyopathy.

5. Use as claimed in claim 3 wherein the acute coronary syndrome to be treated is unstable angina pectoris, non-Q wave myocardial infarction or Q-wave myocardial infarction.

6. Use as claimed in claim 3 wherein the chronic CHF to be treated is secondary to either idiopathic dilated cardiomyopathy (IDCM) and/or coronary artery disease (CAD).

7. Use as claimed in any one of claims 1 to 6 wherein said IVIG has the effect of down regulating immune activation and/or down regulating inflammation processes.

8. Use as claimed in claim 7 wherein the levels or activity of one or more pro-inflammatory cytokines and/or cytokine receptors and/or chemokines and/or chemokine receptors are reduced and/or the levels or activity of one or more anti-inflammatory cytokines and/or cytokine receptors and/or chemokines and/or chemokine receptors are increased.

9. Use as claimed in claim 8 wherein said pro-inflammatory cytokines are IL-1β, TNFα or IL-1, said anti-inflammatory cytokines or receptors are IL-10, IL-1Ra or sTNFRs, said pro-inflammatory chemokines or receptors are MIP-1α, MIP-1β, IL-8, CCR1, CCR2, CCR5, CXCR1, CXCR2 or CX₃CR and said anti-inflammatory chemokine receptors are CCR7 or CXCR5.

10. Use as claimed in any one of claims 1 to 9 wherein cardiac function is improved.

11. Use as claimed in claim 10 wherein ventricular function is increased.

12. Use as claimed in claim 11 wherein left ventricular function is increased.

13. Use as claimed in claim 10 wherein the improvement in cardiac function is one or more of the following: New York Heart Association (NYHA) functional class of heart disease is reduced, exercise capacity is improved, pulmonary capillary wedge pressure is decreased, pulmonary artery pressure is decreased, peak heart rate is increased, peak systolic blood pressure is increased, or mitral velocity deceleration time is increased.

14. Use as claimed in any one of claims 1 to 10 wherein the plasma level of Nt-proANP is reduced.

15. A method of testing whether a patient with a non-viral or non-autoimmune induced heart disorder as defined in any one of claims 1 to 6 is a good candidate for treatment with an immunomodulating agent, comprising analysing a patient's blood sample for up-regulation or elevated levels of pro-inflammatory chemokines, cytokines or their receptors and/or the down-regulation or reduction in levels of anti-inflammatory chemokines, cytokines or their receptors.

16. A method of testing as claimed in claim 18 wherein the cytokines, chemokines, and their receptors are as defined in claim 9.

17. A product comprising (a) intravenous immunoglobulin (IVIG) and (b) a second agent effective for the treatment of heart disorders as a combined preparation for simultaneous, separate or sequential use in the treatment of heart disorders or in improving cardiac function.

18. The product of claim 17 wherein said second agent is selected from the group consisting of diuretics, vasodilators, inotropic drugs such as digoxin, anticoagulants, β blockers, angiotensin II blockers and angiotensin converting enzyme (ACE) inhibitors.

## Patentansprüche

1. Verwendung von intravenösem Immunglobulin (IVIG) bei der Herstellung eines Medikaments zur Verwendung bei der Behandlung nichtviral oder nichtautoimmun induzierter Herzerkrankungen oder nichtviral oder nichtautoimmun assoziierter Phasen von Herzerkrankungen, wobei die Herzerkrankung eine ischämische Herzerkrankung oder eine chronische nichtischämische Kardiomyopathie ist.

2. Verwendung von intravenösem Immunglobulin (IVIG) bei der Herstellung eines Medikaments zur Verwendung bei der Behandlung oder Linderung von Symptomen, die mit Herzerkrankungen oder Phasen davon, wie in Anspruch 1 definiert, assoziiert sind.

3. Verwendung nach Anspruch 1 oder Anspruch 2, wobei die zu behandelnde Herzerkrankung ausgewählt ist unter chronischer CHF, chronischer (stabiler) Angina pectoris und akuten koronaren Syndromen.

4. Verwendung nach einem der Ansprüche 1 bis 3, wobei die Herzerkrankung eine chronische ischämische Kardiomyopathie ist.

5. Verwendung nach Anspruch 3, wobei das zu behandelnde akute koronare Syndrom eine instabile Angina pectoris, ein Nicht-Q-Wellen-Myokardinfarkt oder Q-Wellen-Myokardinfarkt ist.

6. Verwendung nach Anspruch 3, wobei die zu behandelnde chronische CHF sekundär ist zu entweder einer idiopathischen dilatierten Kardiomyopathie (IDCM) und/oder koronarer Herzkrankheit (CAD).

7. Verwendung nach einem der Ansprüche 1 bis 6, wobei das IVIG die Wirkung besitzt, die Immunaktivierung herunterzuregulieren und/oder inflammatorische Vorgänge herunterzuregulieren.

8. Verwendung nach Anspruch 7, wobei die Niveaus oder Aktivität eines oder mehrerer proinflammatorischer Cytokine und/oder Cytokinrezeptoren und/oder Chemokine und/oder Chemokinrezeptoren vermindert sind und/oder die Niveaus oder Aktivität eines oder mehrerer antiinflammatorischer Cytokine und/oder Cytokinrezeptoren und/oder Chemokine und/oder Chemokinrezeptoren erhöht sind.

9. Verwendung nach Anspruch 8, wobei die proinflammatorischen Cytokine IL-1β, TNFα oder IL-1 sind, die antiinflammtorischen Cytokine oder Cytokinrezeptoren IL-10, IL-1Ra oder sTNFRs sind, die proinflammatorischen Chemokine oder Chemokinrezeptoren MIP-1α, MIP-1β, IL-8, CCR1, CCR2, CCR5, CXCR1, CXCR2 oder CX₃CR sind und die antiinflammatorischen Chemokinrezeptoren CCR7 oder CXCR5 sind.

10. Verwendung nach einem der Ansprüche 1 bis 9, wobei die Herzfunktion verbessert ist.

11. Verwendung nach Anspruch 10, wobei die ventrikuläre Funktion verbessert ist.

12. Verwendung nach Anspruch 11, wobei die linksventrikuläre Funktion verbessert ist.

13. Verwendung nach Anspruch 10, wobei die Verbesserung der Herzfunktion sich in einem oder mehreren der folgenden Punkte äußert: Geringere New York Heart Association (NYHA)-Klassifikation für Herzkrankheit, bessere körperliche Belastbarkeit, geringerer Lungenkapillarenverschlussdruck, geringerer Lungenarteriendruck, höhere maximale Herzfrequenz, höherer maximaler systolischer Blutdruck oder längere Verzögerungszeit der Mitralklappengeschwindigkeit.

14. Verwendung nach einem der Ansprüche 1 bis 10, wobei der Plasmaspiegel von Nt-proANP vermindert ist.

15. Verfahren zum Testen, ob ein Patient mit einer nichtviral oder nichtautoimmun induzierten Herzerkrankung, wie in einem der Ansprüche 1 bis 6 definiert, ein guter Kandidat zur Behandlung mit einem immunmodulierenden Agens ist, wobei man eine Blutprobe des Patienten auf hochregulierte oder erhöhte Spiegel von proinflammatorischen Chemokinen, Cytokinen oder ihren Rezeptoren und/oder herunterregulierte oder verminderte Spiegel von antiinflammatorischen Chemokinen, Cytokinen oder ihren Rezeptoren analysiert.

16. Verfahren zum Testen nach Anspruch 18, wobei die Cytokine, Chemokine und ihre Rezeptoren wie in Anspruch 9 definiert sind.

17. Produkt, umfassend (a) intravenöses Immunglobulin (IVIG) und (b) ein zweites Agens mit Wirksamkeit bei der Behandlung von Herzerkrankungen als kombiniertes Präparat für die simultane, getrennte oder aufeinanderfolgende Verwendung bei der Behandlung von Herzerkrankungen oder zur Verbesserung der Herzfunktion.

18. Produkt nach Anspruch 17, wobei das zweite Agens ausgewählt ist unter Diuretika, Vasodilatoren, inotropen Wirkstoffen wie Digoxin, Antikoagulantien, β-Blockern, Angiotensin-II-Blockern und Hemmern des Angiotensin Converting Enzyms (ACE).

## Revendications

1. Utilisation d'une immunoglobuline intraveineuse (IVIG) pour fabriquer un médicament destiné à être utilisé pour le traitement de maladies cardiaques d'origine non virale et non auto-immune ou des phases associées non virales et non auto-immunes de maladies cardiaques, ladite maladie cardiaque étant une maladie cardiaque ischémique ou une cardiomyopathie non ischémique.

2. Utilisation d'une immunoglobuline intraveineuse (IVIG) pour fabriquer un médicament destiné à être utilisé pour le traitement ou le soulagement de symptômes liés aux maladies cardiaques ou à leurs phases telles qu'elles sont définies dans la revendication 1.

3. Utilisation selon la revendication 1 ou la revendication 2, la maladie cardiaque à traiter étant choisie dans le groupe comprenant l'insuffisance cardiaque chronique, l'angine de poitrine chronique (stable) et les syndromes coronariens aigus.

4. Utilisation selon l'une quelconque des revendications 1 à 3, ladite maladie cardiaque étant une cardiomyopathie chronique ischémique.

5. Utilisation selon la revendication 3, le syndrome coronarien aigu à traiter étant l'angine de poitrine instable, l'infarctus du myocarde sans onde Q ou l'infarctus du myocarde à onde Q.

6. Utilisation selon la revendication 3, l'insuffisance cardiaque chronique à traiter étant secondaire à une cardiomyopathie dilatée idiopathique (CMDI) et/ou à une coronaropathie.

7. Utilisation selon l'une quelconque des revendications 1 à 6, ladite IVIG ayant l'effet de réguler à la baisse l'activation immune et/ou de réguler à la baisse les processus d'inflammation.

8. Utilisation selon la revendication 7, les niveaux ou l'activité d'une ou plusieurs cytokines pro-inflammatoires et/ou récepteurs de cytokines et/ou chimiokines et/ou récepteurs de chimiokines étant réduits et/ou les niveaux ou l'activité d'une ou plusieurs cytokines anti-inflammatoires et/ou récepteurs de cytokines et/ou chimiokines et/ou récepteurs de chimiokine sont accrus.

9. Utilisation selon la revendication 8, lesdites cytokines pro-inflammatoires étant l'IL-1β, le TNFα ou l'IL-1, lesdites cytokines anti-inflammatoires ou lesdits récepteurs de cytokines anti-inflammatoires sont l'IL-10, l'IL1Ra ou les sTNFR, lesdites chimiokines pro-inflammatoires ou lesdits récepteurs de chimiokines pro-inflammatoires sont MIP-1α, MIP-1β, IL-8, CCR1, CCR2, CCR5, CXCR1, CXCR2 ou CX₃CR et lesdits récepteurs de chimiokines anti-inflammatoires sont CCR7 ou CXCR5.

10. Utilisation selon l'une quelconque des revendications 1 à 9 dans laquelle la fonction cardiaque est améliorée.

11. Utilisation selon la revendication 10 dans laquelle la fonction ventriculaire est accrue.

12. Utilisation selon la revendication 11 dans laquelle la fonction ventriculaire gauche est accrue.

13. Utilisation selon la revendication 10 dans laquelle l'amélioration de la fonction cardiaque est une ou plusieurs des améliorations suivantes : réduction d'une maladie cardiaque de clase fonctionnelle de la classification de la New York Heart Association (NYHA), amélioration de la capacité d'exercice, diminution de la pression capillaire pulmonaire bloquée, diminution de la pression artérielle pulmonaire, augmentation de la fréquence cardiaque maximale, augmentation de la pression sanguine systolique maximale ou augmentation du temps de décélération de la vitesse mitrale.

14. Utilisation selon l'une quelconque des revendications 1 à 10 dans laquelle le niveau plasmatique de Nt-proANP est réduit.

15. Procédé pour tester si un patient présentant une maladie cardiaque d'origine non virale et non auto-immune telle qu'elle est définie dans l'une quelconque des revendications 1 à 6 est un bon candidat pour un traitement avec un agent immunomodulateur, comprenant l'analyse d'un échantillon de sang du patient pour régulation à la hausse ou niveaux élevés de chimiokines pro-inflammatoires, de cytokines pro-inflammatoires ou de leurs récepteurs et/ou régulation à la baisse ou réduction des niveaux de chimiokines anti-inflammatoires, de cytokines anti-inflammatoires ou de leurs récepteurs.

16. Procédé de test selon la revendication 18 dans lequel les cytokines, les chimiokines et leurs récepteurs sont comme définis dans la revendication 9.

17. Produit comprenant (a) une immunoglobuline intraveineuse (IVIG) et (b) un second agent efficace pour le traitement de maladies cardiaques sous forme de préparation combinée pour utilisation simultanée, séparée ou séquentielle dans le traitement de maladies cardiaques ou pour améliorer la fonction cardiaque.

18. Produit selon la revendication 17 dans lequel ledit second agent est choisi dans le groupe comprenant des diurétiques, des vasodilatateurs, des médicaments inotropes comme la digoxine, des anti-coagulants, des bêtabloquants, des inhibiteurs de l'angiotensine II et des inhibiteurs de l'enzyme de conversion de l'angiotensine (IEC).
